(19) **Europäisches Patentamt / European Patent Office / Office européen des brevets**

(11) **EP 2 690 133 A1**

(12) **EUROPEAN PATENT APPLICATION**
published in accordance with Art. 153(4) EPC

(43) Date of publication:
**29.01.2014 Bulletin 2014/05**

(51) Int Cl.:
*C08L 3/10* (2006.01)          *C08G 81/00* (2006.01)
*C08L 33/04* (2006.01)

(21) Application number: **12761236.4**

(22) Date of filing: **21.03.2012**

(86) International application number:
**PCT/JP2012/057089**

(87) International publication number:
**WO 2012/128264 (27.09.2012 Gazette 2012/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.03.2011  JP 2011065103**

(71) Applicant: **Sanyo Chemical Industries, Ltd.**
**Kyoto-shi**
**Kyoto 605-0995 (JP)**

(72) Inventors:
• **UEDA, Yusuke**
  **Kyoto-shi**
  **Kyoto 605-0995 (JP)**
• **KANDA, Yoichi**
  **Kyoto-shi**
  **Kyoto 605-0995 (JP)**

(74) Representative: **Vossius & Partner**
**Siebertstrasse 4**
**81675 München (DE)**

(54) **AQUEOUS-LIQUID-ABSORBABLE RESIN, AQUEOUS-LIQUID-ABSORBABLE COMPOSITION, AND ABSORBER MATERIAL AND ABSORBABLE OBJECT EACH PRODUCED USING SAME**

(57)     An absorbable resin, for which a large amount of a plant-derived raw material can be used, and which has an excellent capability of absorbing an aqueous liquid, and has a good gel modulus and therefore can exhibit an excellent absorbing capability under pressurized conditions; and an absorbable composition. An absorbable resin produced by binding an oxidized polysaccharide (A1) having a carboxyl group which may be neutralized with a neutralizing agent and/or a crosslinked product thereof (A2) to a poly(meth)acrylic acid (B1) in which at least one carboxyl group may be neutralized with a neu-
tralizing agent and/or a crosslinked product thereof (B2); or an absorbable composition produced by mixing the component (A1) and/or the component (A2) with the component (B1) and/or the component (B2). The component (A1) has a weight average molecular weight of 2,000-10,000,000. The component (A1) has an acid value of 65-850 mgKOH/g. When the component (A1) has a carboxyl group which is neutralized with a neutralizing agent, the acid value of the component (A1) before the neutralization is 65-850 mgKOH/g.

**Description**

TECHNICAL FIELD

The present invention relates to an aqueous liquid absorbent resin, an aqueous liquid absorbent composition, and an aqueous liquid absorber and an aqueous liquid absorbent article each using the same.

BACKGROUND ART

Conventionally, as a particulate absorbing agent having an absorption ability for an aqueous liquid, a hydrophilic crosslinked polymer called a water absorbent resin has been known. Such an absorbent resin includes, for example, various known resins such as crosslinked polyacrylic acid salt, crosslinked copolymer of an acrylic acid or a salt thereof and another monomer, crosslinked isobutylene-maleic anhydride copolymer, polyvinyl alcohol-(meth)acrylic acid copolymer, modified polyethylene oxide and modified polyvinyl alcohol, and is used mainly for hygienic materials such as disposable diaper and sanitary product.
In recent years, use of a plant-derived raw material for a water absorbent resin is being attempted, and a starch-acrylic acid salt copolymer is known. As described in Non-Patent Document 1, when the starch content is small, the water absorptivity is enhanced, but when a large amount of starch is mixed, water absorptivity is disadvantageously reduced. This is considered to result because the starch has no ionic group and does not exhibit water absorptivity by itself. Patent Document 1 describes a modified starch composition as a water absorbent powder obtained by slightly oxidizing starch to allow from 0.05 to 0.5 wt% of carboxyl group to be present and then crosslinking the starch. The powder obtained by this method has a problem that water absorption ability is low due to a small amount of carboxyl group and also, the water absorption ability in a pressurized state is bad because the elastic modulus of the starch is low.

PRIOR ART DOCUMENTS

PATENT DOCUMENTS

Patent Document 1: EP-A-0489424

NON-PATENT DOCUMENTS

Non-Patent Document 1: Masuda, Chemical Economy & Engineering Review; November 1983, page 19 (No. 173)

SUMMARY OF THE INVENTION

PROBLEMS TO BE SOLVED BY THE INVENTION

The present invention has been made by taking into account these problems, and an object of the present invention is to provide an absorbent resin and an absorbent composition each using a plant-derived raw material in a large proportion and being excellent in the aqueous liquid absorption ability and thanks to good gel elastic modulus, also excellent in the aqueous liquid absorption ability in a pressurized state.

MEANS FOR SOLVING THE PROBLEMS

As a result of intensive studies to attain the object above, the present inventors have achieved the present invention. That is, the present invention includes:

(C) an aqueous liquid absorbent resin (hereinafter, sometimes simply referred to as absorbent resin) formed by binding (A1) a polysaccharide oxide having a carboxyl group which may be neutralized with a neutralizer, and/or (A2) a crosslinked product thereof, to (B1) a poly(meth)acrylic acid in which at least one carboxyl group may be neutralized with a neutralizer, and/or (B2) a crosslinked product thereof, wherein a weight average molecular weight of the (A1) is from 2,000 to 10,000,000, an acid value of the (A1) is from 65 to 850 mgKOH/g when the (A1) does not have a carboxyl group neutralized with a neutralizer, and an acid value of the (A1) before neutralization is from 65 to 850 mgKOH/g when the (A1) has a carboxyl group neutralized with a neutralizer;
(P-1) an absorbent resin particle comprising the absorbent resin (C) and (E) a hydrophobic substance, wherein the (E) is a compound having at least one monovalent aliphatic hydrocarbon group with a carbon number of 8 to 26 and having at least one functional group capable of forming a hydrogen bond with a carboxyl group, and based on the weight of the absorbent resin, the (E) is present in an amount of 0.01 to 10.0 wt% in the inside of the absorbent resin particle and present in an amount of 0.001 to 1.0 wt% on the surface of the absorbent resin particle;

(D) an aqueous liquid absorbent composition (hereinafter, sometimes simply referred to as absorbent composition) comprising: (A1) a polysaccharide oxide having a carboxyl group which may be neutralized with a neutralizer, and/or (A2) a crosslinked product thereof; and (B1) a poly(meth)acrylic acid in which at least one carboxyl group may be neutralized with a neutralizer, and/or (B2) a crosslinked product thereof, wherein a weight average molecular weight of the (A1) is from 2,000 to 10,000,000, an acid value of the (A1) is from 65 to 850 mgKOH/g when the (A1) does not have a carboxyl group neutralized with a neutralizer, and an acid value of the (A1) before neutralization is from 65 to 850 mgKOH/g when the (A1) has a carboxyl group neutralized with a neutralizer;

(P-2) an absorbent composition particle comprising: the absorbent composition (D) and (E) a hydrophobic substance, wherein the (E) is a compound having at least one monovalent aliphatic hydrocarbon group with a carbon number of 8 to 26 and having at least one functional group capable of forming a hydrogen bond with a carboxyl group, and based on the weight of the absorbent composition particle, the (E) is present in an amount of 0.01 to 10.0 wt% in the inside of the absorbent composition particle and present in an amount of 0.001 to 1.0 wt% on the surface of the absorbent composition particle;

an absorber formed using the absorbent resin (C), the  absorbent composition (D), the absorbent resin particle (P-1) or the absorbent composition particle (P-2) and at least one member selected from the group consisting of a fiber, a nonwoven fabric and a woven fabric; and an absorbent article formed using the absorber.

## ADVANTAGES OF THE INVENTION

The absorbent resin and the absorbent composition of the present invention each uses a plant-derived raw material in a large proportion and is excellent in the aqueous liquid absorption ability and gel elastic modulus, whereby an absorption ability in a pressurized state is also excellent.

## BRIEF DESCRIPTION OF THE DRAWINGS

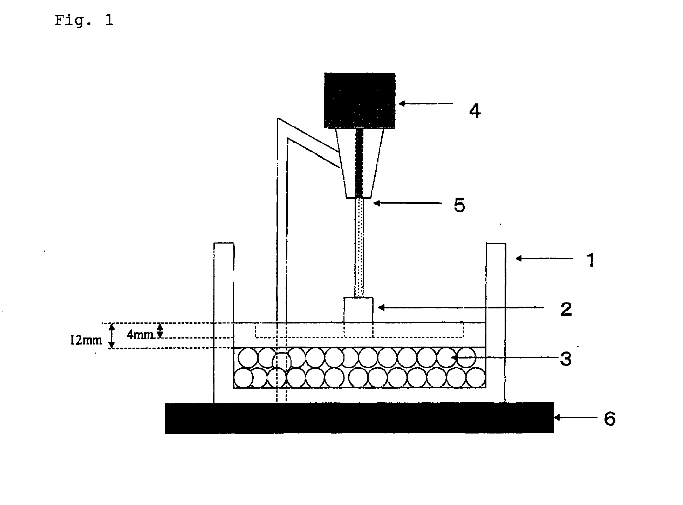

[Fig. 1] Fig. 1 is a front cross-sectional view schematically illustrating a whole device for measuring the absorption amount by a swelled volume measuring method.

## MODE FOR CARRYING OUT THE INVENTION

The absorbent resin (C) of the present invention is an absorbent resin formed by binding (A1) a polysaccharide oxide having a carboxyl group which may be neutralized with a neutralizer (hereinafter, sometimes simply referred to as polysaccharide oxide (A1) or the (A1)), and/or (A2) a crosslinked product thereof (hereinafter, sometimes simply referred to as crosslinked product (A2) or the (A2)), to (B1) a poly(meth)acrylic acid in which at least one  carboxyl group may be neutralized with a neutralizer (hereinafter, sometimes simply referred to as poly (meth) acrylic acid (B1) or the (B1)), and/or (B2) a crosslinked product thereof (hereinafter, sometimes simply referred to as crosslinked product (B2) or the (B2)), wherein the weight average molecular weight of the (A1) is from 2,000 to 10,000,000, the acid value of the (A1) is from 65 to 850 mgKOH/g when the (A1) does not have a carboxyl group neutralized with a neutralizer, and the acid value of the (A1) before neutralization is from 65 to 850 mgKOH/g when the (A1) has a carboxyl group neutralized with a neutralizer.

The polysaccharide oxide (A1) for use in the present invention includes oxides of starch, cellulose, glycogen, chitin, chitosan, agarose, carrageenan, heparin, hyaluronic acid, pectin, xyloglucan and the like, and these oxides having a carboxyl group which may be neutralized with a neutralizer. In the case of having a plurality of carboxyl groups, at least a part thereof may be neutralized. Among these, in view of reactivity, a starch oxide, a cellulose oxide, and these oxides having a carboxyl group which may be neutralized with a neutralizer, are preferred. As for the polysaccharide oxide (A1), one may be used alone, or two or more may be used in combination.

Examples of the starch used for the starch oxide include corn starch, potato starch, tapioca starch, wheat starch, sweet potato starch, and rice starch. In addition, modified starches using these starches as a raw material, such as water-soluble starch, oxidized starch, carboxylated  starch, phosphorylated starch, cationized starch, aldehyded starch, acetylated starch, alkyl etherified starch, hydroxy etherified starch, allyl etherified starch, and vinyl etherified starch, may be also used. Examples of the water-soluble starch include a hydrolyzed starch obtained by acid hydrolysis. Examples of the oxidized starch include commercially available general oxidized starches treated with hypochlorite or the like. The commercially available general oxidized starch is a starch where a part of hydroxyl groups in a glucopyranose as a constituent unit of the starch are carboxylated or aldehyded.

Examples of the cellulose used for the cellulose oxide include cotton, wood-derived pulp, bacteria cellulose, lignocellulose, regenerated cellulose (for example, Cellophane and regenerated fiber), and microcrystalline cellulose.

As the method to obtain the polysaccharide oxide (A1), a generally known method may be used. For example, there may be used a method of performing oxidation by using a catalyst based on a noble metal such as palladium and an oxygen-containing gas described in JP-A-62-247837, and a method of performing oxidation by using a manganese oxide or a salt thereof described in JP-A-10-195101.

The acid value of the polysaccharide oxide (A1) is from 65 to 850 mgKOH/g, and in view of absorption ability, is preferably from 300 to 850 mgKOH/g. If the acid value is less than 65 mgKOH/g, the ionic group density of the absorbent resin formed becomes low, giving rise to bad absorption ability, whereas if the acid value exceeds 850 mgKOH/g, oxidation proceeds to cause generation of many low-molecular-weight materials, as a result, the gel elastic modulus is reduced. The acid value can be measured by the following method.

<Measurement Method of Acid Value>

Into a 300 ml-volume beaker, 1.00 g of a non-neutralized polysaccharide oxide is precisely weighed and after adding pure water to make 100 g, dissolved with stirring at 95°C for 15 minutes to prepare a measurement solution. The measurement solution is first titrated to pH 10 with an aqueous 1/50 N KOH solution and then titrated to pH 2.7 with an aqueous 1/20 N HCl solution. Also, as a blank, 100 g of pure water is titrated by the same method. The amount of the aqueous 1/20 N HCl solution necessary for titration from pH 10 to pH 2.7 in the blank is subtracted from the amount of the aqueous 1/20 N HCl solution necessary for titration from pH 10 to pH 2.7 in the measurement solution having dissolved therein 1.00 g of a polysaccharide oxide, and from the obtained value, the acid value is calculated.

Incidentally, in the case where a part or all of carboxyl groups in the polysaccharide oxide (A1) measured for the acid value are neutralized with a neutralizer to make a salt form, an aqueous 1 wt% solution of the polysaccharide oxide (A1) is prepared, mixed with a cation exchange resin (DOWEX50W-X8) and stirred for 15 minutes, thereby effecting cation exchange and conversion to a non-neutralization type polysaccharide oxide, and thereafter, the acid value is measured in the same manner as above.

The weight average molecular weight (hereinafter, simply referred to as Mw) of the polysaccharide oxide (A1) is from 2,000 to 10,000,000, and in view of resin strength and absorption ability, preferably from 5,000 to 5,000,000, more preferably from 10,000 to 1,000,000. If the weight average molecular weight is less than 2,000, elution to the absorbing liquid is increased and in turn, the absorption ability is impaired, whereas if it exceeds 10,000,000, the absorption ability is reduced.

The Mw of the (A1) can be measured by the gel permeation chromatography (GPC) method under the following conditions:

Solvent: an aqueous 30 vol% methanol solution containing 0.5 wt% of sodium acetate
Sample concentration: 2.5 mg/ml
Column used: Guardcolumn PWXL + TSKgel G6000 PWXL + TSKgel G3000 PWXL, manufactured by Tosoh Corporation
Column temperature: 40°C

Examples of the neutralizer for neutralizing a carboxyl group contained in the polysaccharide oxide (A1) include ammonia, an amine compound having a carbon number of 1 to 20, and an alkali metal hydroxide (such as sodium hydroxide, potassium hydroxide and lithium hydroxide).

The amine compound having a carbon number of 1 to 20 includes a primary amine such as monomethylamine, monoethylamine, monobutylamine and monoethanolamine, a secondary amine such as dimethylamine, diethylamine, dibutylamine, diethanolamine, diisopropanolamine and methylpropanolamine, and a tertiary amine such as trimethylamine, triethylamine, dimethylethylamine, dimethylmonoethanolamine and triethanolamine.

Among these neutralizers, from the standpoint of suppressing coloration after neutralization, an alkali metal hydroxide is preferred. As for the neutralizer, one may be used alone, or two or more may be used in combination.

The method for obtaining (A2) a crosslinked product of the polysaccharide oxide (A1) include a method of crosslinking the (A1), a method using a crosslinked polysaccharide as the raw material polysaccharide used for oxidation, and a method using these methods in combination.

The method of crosslinking the (A1) includes a method of performing the crosslinking by using the following (k1) to (k6):

(1) (k1) a polyhydric (preferably from dihydric to tetrahydric) alcohol having a carbon number of 2 to 6 (such as ethylene glycol, diethylene glycol, triethylene glycol, 1,3-propanediol, dipropylene glycol, glycerin, diglycerin, 1,4-butanediol, 1,5-pentanediol, 1,6-hexanediol and sorbitol);

(2) (k2) a polyvalent (preferably from divalent to tetravalent) glycidyl ether having a carbon number of 8 to 21 [such as ethylene glycol diglycidyl ether, polyethylene glycol (polymerization degree: from 2 to 4) diglycidyl ether, glycerin polyglycidyl ether, diglycerin polyglycidyl ether, propylene glycol diglycidyl ether, polypropylene glycol (polymerization degree: from 2 to 3) diglycidyl ether, sorbitol polyglycidyl ether, neopentyl glycol diglycidyl ether and 1,6-hexanediol diglycidyl ether];

(3) (k3) a polyvalent (preferably from divalent to tetravalent) amine having a carbon number of 2 to 6 (such as ethylenediamine, diethylenetriamine, triethylenetetramine and polyethyleneimine);

(4) (k4) an alkanolamine having a carbon number of 2 to 8 (such as monoethanolamine, diethanolamine, monopropanolamine and dibutanolamine);

(5) (k5) a polyvalent (preferably from divalent to tetravalent) aziridine compound having a carbon number of 6 to 12 [such as 2,2-bishydroxymethylbutanol-tris[3-(1-aziridinyl)propionate], 4,4'-bis(ethyleneiminocarbonylamino)diphenylmethane and 1,6-bis(ethyleneiminocarbonylamino)hexane]; and

(6) (k6) an alkylene carbonate having a carbon number of 3 to 4 (such as 1,3-dioxolan-2-one, 4-methyl-1,3-dioxolan-2-one and 1,3-dioxan-2-one).

Among these, in view of the crosslinking density, (k1), (k2), (k3) and (k4) are preferred, (k2) and (k3) are more preferred, and (k2) is still more preferred. As for (k1) to (k6), one may be used alone, or two or more may be used in combination.

The polysaccharide can be crosslinked by an arbitrary known method and may by crosslinked using a crosslinking agent or may be crosslinked by radiation (radiation such as $\gamma$-ray, $\chi$-ray or electron beam) and/or heat.

Examples of the crosslinking agent used for the crosslinking of the polysaccharide include a methylol group-containing urea-based or melamine-based compound (such as dimethylolurea, trimethylolmelamine, dimethylolethyleneurea and dimethyloldihydroxyethyleneurea), a polycarboxylic acid (such as citric acid, tricarballylic acid and 1,2,3,4-butanetetracarboxylic acid), and the above-described (k1) to (k5). One of these may be used alone, or two or more thereof may be used in combination.

The conditions for the crosslinking reaction of the (A1) vary depending on the crosslinking agent used, but the conditions (for example, from 50 to 100°C, and from 10 minutes to 2 hours) usually employed for the reaction between a functional group contained in a polysaccharide or the (A1) and a functional group contained in the crosslinking agent may be applied.

The production method of (B1) a poly(meth)acrylic acid for use in the present invention is not particularly limited and includes, for example, a method of subjecting a (meth)acrylic acid monomer which may be neutralized with a neutralizer, to aqueous solution polymerization, suspension polymerization or reverse phase suspension polymerization by using a radical polymerization catalyst. A method of irradiating the monomer with radiation, an electron beam or an ultraviolet ray to initiate the polymerization may be also employed. Among these, aqueous solution polymerization is preferred, because an organic solvent or the like need not be used and this is advantageous in view of the production cost. From the standpoint that an absorbent resin having a large water-retention amount and a small amount of water-soluble components is obtained and the temperature control during polymerization is unnecessary, an aqueous solution adiabatic polymerization method is morc preferred.

In the present invention, the polymerization conditions are not particularly limited, and, for example, the polymerization initiation temperature may be varied according to the kind of the catalyst but is usually from 0 to 100°C, preferably from 5 to 80°C.

As for the polymerization catalyst used when performing polymerization by using a radical polymerization catalyst, a conventionally known catalyst may be used, and examples thereof include an azo compound (such as azobisisobutyronitrile, azobiscyanovaleric acid and 2,2'-azobis(2-amidinopropane)hydrochloride), an inorganic peroxide (such as hydrogen peroxide, ammonium persulfate, potassium persulfate and sodium persulfate), an organic peroxide (such as benzoyl peroxide, di-tert-butyl peroxide, cumene hydroperoxide, succinic peroxide and di(2-ethoxyethyl)peroxydicarbonate), a cerium compound (such as ammonium cerium(IV) nitrate and ammonium hexanitratocerium(IV)), and a redox catalyst (comprising a combination of a reducing agent such as alkali metal sulfite or bisulfite, ammonium sulfite, ammonium bisulfite and ascorbic acid, and an oxidizing agent such as alkali metal persulfate, ammonium persulfate, hydrogen peroxide and organic peroxide). One of these catalysts may be used alone, or two or more thereof may be used in combination.

The amount of the radical polymerization catalyst used is usually from 0.0005 to 5 wt%, preferably from 0.001 to 2 wt%, based on the weight of the radical polymerizable monomer.

The method for neutralization using a neutralizer includes a method of using a (meth)acrylic acid monomer after neutralizing the monomer, a method of polymerizing a (meth)acrylic acid monomer and then neutralizing the polymer, and a method using these methods in combination.

Examples of the neutralizer for neutralizing a carboxyl group contained in the (B1) are the same as those of the neutralizer for neutralizing a carboxyl group contained in the polysaccharide oxide (A1), and preferred examples are also the same.

The method for obtaining the crosslinked product (B2) includes, for example, a method of performing crosslinking by using (k1) to (k5), and a method of performing crosslinking by using (k7) a compound having a radical polymerizable double bond. As for each of (k1) to (k5) and (k7), one may be used alone, or two or more may be used in combination.

The (k7) compound having a radical polymerizable double bond includes (k71) a compound having two or more radical polymerizable double bonds, and (k72) a compound having one functional group capable of reacting with a carboxyl group or a neutralized salt thereof and having at least one radical polymerizable double bond.

Examples of the (k71) compound having two or more radical polymerizable double bonds include bis(meth) acrylamides such as N,N'-methylenebisacrylamide; poly(meth)acrylates or poly(meth)allyl ethers of a polyhydric alcohol such as (poly)alkylene glycol, trimethylolpropane, glycerin, pentaerythritol and sorbitol; divinyl compounds such as divinylbenzene; and allyloxyalkanes such as tetraallyloxyethane and pentaerythritol triallyl ether.

Examples of the (k72) compound having one functional group capable of reacting with a carboxyl group or a neutralized salt thereof and having at least one radical polymerizable double bond include a hydroxyl group-containing radical polymerizable monomer such as hydroxyethyl (meth)acrylate and N-methylol(meth)acrylamide, and an epoxy group-containing radical polymerizable monomer such as glycidyl (meth)acrylate.

The conditions for the crosslinking reaction of the (B1) when using (k1) to (k5) as a crosslinking agent may vary depending on the crosslinking agent used, but the conditions (for example, from 50 to 100°C, and from 10 minutes to 2 hours) usually employed for the reaction between a carboxyl group contained in the (B1) and a functional group contained in the crosslinking agent may be applied. Also, in the case of using (k7) as a crosslinking agent, for example, a (meth)acrylic acid monomer and (k7) are mixed and polymerized under normal conditions (for example, from 0 to 100°C, and from 1 to 10 hours) and when (k72) is used, reaction is further performed under the conditions (for example, from 50 to 100°C, and from 10 minutes to 2 hours) usually employed for the reaction between a carboxyl group contained in (B1) and a functional group contained in (k72), whereby (B2) a crosslinked product of (B1) is obtained.

The absorbent resin (C) for use in the present invention is formed by binding (A1) a polysaccharide oxide and/or (A2) a crosslinked product thereof, to (B1) a poly(meth)acrylic acid and/or (B2) a crosslinked product thereof.

Examples of the method for binding the (A1) and/or (A2) to the (B1) and/or (B2) include a method of binding these by using a binder, and a method of graft-polymerizing (B1) and/or (B2) to (A1) and/or (A2).

Examples of the binder include the (k1) polyhydric alcohol having a carbon number of 2 to 6, the (k2) polyvalent glycidyl ether having a carbon number of 8 to 21, the (k3) polyvalent amine having a carbon number of 2 to 6, the (k4) alkanolamine having a carbon number of 2 to 8, and the (k5) polyvalent aziridine compound having a carbon number of 6 to 12. As for (k1) to (k5), one may be used alone, or two or more may be used in combination.

Among these, in view of the binding density, (k1), (k2) and (k3) are preferred, (k2) and (k3) are more preferred, and (k2) is still more preferred.

In view of the number of bonds and the water-retention amount, the amount of the binder used is preferably from 0.01 to 10 wt%, more preferably from 0.02 to 5 wt%, based on the weight of the absorbent resin (C).

After mixing the binder, the binding reaction is completed by overheating. The heating may be performed in a mixing apparatus or in a heated dryer. In view of prevention of thermal decomposition and the water-retention amount, the heating temperature is preferably from 30 to 250°C, more preferably from 50 to 180°C. The heating time may be varied according to the heating temperature, but in view of the reaction ratio of binding reaction and the water-retention amount, the heating time is preferably from 5 to 180 minutes, more preferably from 15 to 90 minutes.

After mixing the binder, a known alkali agent may be added so as to accelerate the binding reaction. Incidentally, this mixing with alkali is differentiated from the neutralization with alkali in the production process of a normal water absorbent resin but can be performed in the same manner as in the neutralization step. Accordingly, in the case of performing neutralization, the neutralization and the alkali addition may be performed at the same time.

As for the alkali agent, known alkali agents (for example, Japanese Patent No. 3,205,168) may be used. Among these, in view of water absorption ability, lithium hydroxide, sodium hydroxide and potassium hydroxide are preferred, and sodium hydroxide is more preferred. One alkali agent may be used alone, or two or more alkali agents may be used in combination.

The method for graft-polymerizing the (B1) and/or (B2) to the (A1) and/or (A2) is not particularly limited, and examples thereof include a method of performing the reaction of (B1) and/or (B2) in the presence of (A1) and/or (A2) by using an inorganic peroxide, an organic peroxide, a cerium compound or a redox catalyst as the radical polymerization catalyst.

Neutralization of a carboxyl group contained in the (A1), (A2), (B1) and (B2) of the absorbent resin (C) by using a neutralizer may be performed before binding or after binding and obtaining (C).

In view of irritation to skin or the like, the neutralization ratio is preferably less than 85%, more preferably from 65 to 75%, based on the total amount of carboxyl groups contained in the (B1) and (B2) of the absorbent resin (C).

In view of irritation to skin or the like, the neutralization ratio is preferably from 65 to 75% based on the total amount of carboxyl groups contained in the (A1), (A2), (B1) and (B2) of the absorbent resin (C).

From the standpoint of satisfying both the absorption ability and the gel elastic modulus, the total amount of the (A1) and (A2) bound in the absorbent resin (C) is preferably from 30 to 90 wt%, more preferably from 35 to 50%, based on the weight of the absorbent resin.

The absorbent composition (D) of the present invention contains, as essential components, the polysaccharide oxide (A1) and/or the crosslinked product thereof (A2), and the poly(Meth)acrylic acid (B1) and/or the crosslinked product thereof (B2).

The (D) may be obtained by mixing the (A1) and/or (A2) with the (B1) and/or (B2). The mixing method is not particularly limited and includes, for example, a method of mixing an aqueous solution of (A1) and/or a hydrous gel of (A2) with an aqueous solution of (B1) and/or a hydrous gel of (B2) by means of a kneader, a universal mixer, a single-screw or twin-screw melt extruder, a mincing machine, a meat chopper or the like. Among these, a universal mixer, a single-screw or a twin-screw melt extruder, and a mincing machine are preferred, and a single-screw or twin-screw melt extruder and a mincing machine are more preferred.

The (D) may be also obtained by dry-blending the later-described particle composed of the (A1) and/or (A2) and the resin particle composed of the (B1) and/or (B2).

The absorbent composition (D) may further contain the absorbent resin (C). By virtue of containing (C), it is more facilitated to satisfy both absorption ability and higher gel elastic modulus.

Neutralization of a carboxyl group contained in the (A1), (A2), (B1) and (B2) of the absorbent composition (D) may be preformed before mixing or after mixing and obtaining (D).

In view of irritation to skin or the like, the neutralization ratio is preferably less than 85%, more preferably from 65 to 75%, based on the total amount of carboxyl groups contained in the (B1) and (B2) of the absorbent composition (D).

In view of irritation to skin or the like, the neutralization ratio is preferably from 65 to 75% based on the total amount of carboxyl groups contained in the (A1), (A2), (B1) and (B2) of the absorbent composition (D).

From the standpoint of satisfying both the absorption ability and the gel elastic modulus, the total amount of the (A1) and (A2) contained in the absorbent composition (D) and (A1) and (A2) bound in the absorbent resin (C) contained in (D) is preferably from 30 to 90 wt%, more preferably from 35 to 50 wt%, based on the weight of the absorbent resin.

In the absorbent resin (C) and the absorbent composition (D) of the present invention, conventionally known additives (such as antiseptic, fungicide, antimicrobial, antioxidant, ultraviolet absorbing agent, colorant, fragrance, deodorant, inorganic powder and organic fibrous material) described, for example, in JP-A-2003-225565 and JP-A-2006-131767 may be added. As for the additive, one may be used alone, or two or more may be used in combination.

Water used for the production of the absorbent resin (C) and the absorbent composition (D) is removed by heating/drying in a rotary dryer, a puddle dryer, a Nautor-type dryer, a rotary kiln or the like, whereby (C) and (D) as a solid are obtained.

The shapes of the absorbent resin (C) and the absorbent composition (D) of the present invention may be arbitrarily set according to use, but in the case of use as a hygienic material such as disposable diaper and sanitary product, a particulate form is preferred. The method for making (C) and (D) into particulate form is not particularly limited, and examples thereof include a method of performing pulverization, particle size adjustment or the like by a known method.

The method for pulverization is not particularly limited, and a normal pulverization apparatus (for example, a hammer pulverizer, an impact pulverizer, a roll pulverizer, and a jet stream pulverizer) and the like may be used. The particle size of the particles after pulverization can be adjusted, if desired, by sieving or the like.

In the case of sieving the particles as needed, the weight average particle diameters of the absorbent resin (C) particles and the absorbent composition (D) particles are, in view of absorption ability, preferably from 100 to 800 $\mu$m, more preferably from 200 to 700 $\mu$m, still more preferably from 250 to 600 $\mu$m, yet still more preferably from 300 to 500 $\mu$m, and most preferably from 350 to 450 $\mu$m.

The weight average particle diameter is measured using a Ro-Tap test sieve shaker and standard sieves (JIS Z8801-1:2006) by the method described in Perrys Chemical Engineer's Handbook, 6th edition (MacGraw-Hill Book Company, 1984, page 21). That is, JIS standard sieves are superposed in order of, beginning from the top, 1,000 $\mu$m, 850 $\mu$m, 710 $\mu$m, 500 $\mu$m, 425 $\mu$m, 355 $\mu$m, 250 $\mu$m, 150 $\mu$m, 125 $\mu$m, 75 $\mu$m, and 45 $\mu$m, and a receiving tray is placed at the lowermost part. About 50 g of the measurement particle is put in the topmost sieve and shaken for 5 minutes by using the Ro-Tap test sieve shaker. The weight of the measurement particle on each of the sieves and receiving tray is measured, and by taking the total thereof as 100 wt%, the weight fraction of the particle on each sieve is determined. After plotting the obtained value on a logarithmic probability paper [the sieve opening (particle diameter) as abscissa and the weight fraction as ordinate], a line connecting individual plots is drawn, and a particle diameter corresponding to 50 wt% of the weight fraction is determined. This particle diameter is defined as the weight average particle diameter.

A smaller content of fine particle leads to a higher absorption ability, and therefore, the content of fine particles of 106 $\mu$m or less (preferably 150 $\mu$m or less) in all particles is preferably 3 wt% or less, more preferably 1 wt% or less.

The content of fine particles can be determined using the graph plotting the weight fraction with respect to the sieve opening, which is created when determining the weight average particle diameter above.

The shapes of the absorbent resin (C) particle and the absorbent composition (D) particle, which are obtained by the pulverization method above, are not particularly limited, and examples thereof include an indeterminate crushed shape, a scale shape, a pearl shape, and a rice grain shape. Among these, an indeterminate crushed shape is preferred, because good entangling with a fibrous material in the application such as disposable diaper is ensured and the fear of falling off from the fibrous material is eliminated.

The water-retention amounts (g/g) of the absorbent resin (C) particle and the absorbent composition (D) particle of the present invention are, in view of skin irritation resistance of the absorbent article, preferably from 28 to 45, more preferably from 32 to 40, still more preferably from 34 to 38. The water-retention amount is measured by the following method.

<Measurement Method of Water-Retention Amount>

A measurement sample (1.00 g) is put in a teabag (length: 20 cm, width: 10 cm) formed of a nylon net with a mesh-opening of 63 $\mu$m (JIS Z8801-1:2006) and after dipping in 1,000 ml of physiological saline (salt concentration: 0.9 wt%) with no stirring for 1 hour, the teabag is pulled up and hung for 15 minutes to effect draining. Thereafter, the sample with the teabag is put in a centrifuge and dehydrated by centrifugation at 150 G for 90 seconds to remove excess physiological saline, and the weight (h1) including the teabag is measured. The weight (h2) of the teabag after dehydration by centrifugation is measured in the same manner as above except for not using the measurement sample, and the water-retention amount is determined according to the following formula. Incidentally, the temperatures of the physiological saline used and the measurement atmosphere are 25*C$\pm$2*C.

$$\texttt{Water-retention amount (g/g) = (h1)-(h2)}$$

The gel elastic modulus (N/m$^2$) of the 30-fold swelled gel obtained by causing 30 parts by weight of an artificial urine to be absorbed into 1 part by weight of the absorbent resin (C) particle or the absorbent composition (D) particle of the present invention is preferably from 2,000 to 3,000, more preferably from 2,025 to 2,950, still more preferably from 2,050 to 2,900, and most preferably from 2,075 to 2,850. Within this range, a further excellent leakage resistance is exhibited on application to an absorbent article. Here, the gel elastic modulus (N/m$^2$) is a value determined by the following measurement method.

<Measurement Method of Gel Elastic Modulus>

In a 100 ml-volume beaker (inner diameter: 5 cm), 60.0 g of artificial urine [a mixture of 200 parts by weight of urea, 80 parts by weight of sodium chloride, 8 parts by weight of magnesium sulfate (7 hydrate), 3 parts by weight of calcium chloride (dihydrate), 2 parts by weight of ferric sulfate (7 hydrate), 9,704 parts by weight of ion-exchange water] was weighed, and in the same manner as the operation described in JIS K7224-1996, 2.0 g of the measurement sample is precisely weighed and charged into the beaker above to prepare a 30-fold swelled gel. The beaker containing the 30-fold swelled gel is allowed to stand in an atmosphere of 40$\pm$2°C for 3 hours and further in an atmosphere of 25$\pm$2°C for 0.5 hours, and thereafter, the gel elastic modulus of the 30-fold swelled gel is measured using a curd meter (for example, Curd-Meter MAX ME-500 manufactured by Itec Techno Engineering K.K.). Here, the conditions for measurement by the curd meter are as follows.

Pressure-sensitive shaft: 8 mm
Spring: for 100 g
Load: 100 g
Elevation rate: 1 inch/7 seconds
Test property: breakage
Measurement time: 6 seconds
Measurement atmosphere temperature: 25$\pm$2°C

The gel elastic modulus is further enhanced by applying a crosslinking treatment to the surfaces of the absorbent resin (C) particle and the absorbent composition (D) particle.

Examples of the crosslinking agent used for the surface crosslinking treatment (hereinafter, sometimes referred to as surface crosslinking agent) include the (k1) polyhydric alcohol having a carbon number of 2 to 6, the (k2) polyvalent glycidyl ether having a carbon number of 8 to 21, the (k3) polyvalent amine having a carbon number of 2 to 6, the (k4) alkanolamine having a carbon number of 2 to 8, the (k5) polyvalent aziridine compound having a carbon number of 6 to 12, polyvalent organic isocyanates compound described in JP-A-59-189103, silane coupling agents described in JP-A-61-211305 and JP-A-61-252212, and polyvalent metals described in JP-A-51-136588 and JP-A-61-257235. In this connection, hereinafter, the crosslinking agent for obtaining the (A2) and (B2) is sometimes referred to as an internal crosslinking agent.

Among these, for example, in view of absorption performance, (k2), (k3) and the silane coupling agents are preferred, (k2) and the silane coupling agents are more preferred, and (k2) is still more preferred.

In the case of performing the surface crosslinking treatment, from the standpoint of absorption performance, biodegradability and the like, the amount of the crosslinking agent used is preferably from 0.001 to 3 wt%, more preferably from 0.005 to 2 wt%, still more preferably from 0.01 to 1 wt%, based on the weight of the absorbent resin (C) or absorbent composition (D) before surface crosslinking.

The surface crosslinking treatment can be performed by spraying a mixed solution of the surface crosslinking agent above, water and a solvent on the surface of the absorbent resin (C) or absorbent composition (D) particle and allowing a reaction to proceed under heating. The reaction temperature is usually from 100 to 230°C, preferably from 120 to 160°C. The reaction time may be varied according to the reaction temperature but is usually from 3 to 60 minutes, preferably from 10 to 40 minutes. The particulate absorbent resin and/or absorbent composition obtained by thus performing surface crosslinking may be further surface-crosslinked by a different crosslinking agent.

By incorporating (E) a hydrophobic substance into the absorbent resin (C) particle and the absorbent composition (D) particle, as described in JP-A-2011-252088, the absorption rate pattern can be controlled (the rate is slow in the initial stage, moderate in the middle state, and fast in the late stage) and the leakage resistance is enhanced, so that (P-1) an absorbent resin particle and (P-2) an absorbent composition particle each suitable for an absorbent article (for example, a hygienic material such as disposable diaper and sanitary product) free from a problem of skin irritation can be obtained.

The hydrophobic substance (E) is a compound having at least one monovalent aliphatic hydrocarbon group with a carbon number of 8 to 26 and at least one functional group capable of forming a hydrogen bond with a carboxyl group and is present in a specific amount in the inside of the absorbent resin particle (P-1) and the absorbent composition particle (P-2) and present in a specific amount on the surface of (P-1) and (P-2). This is described later.

Examples of the monovalent aliphatic hydrocarbon group with a carbon number of 8 to 26 include an octyl group, a decyl group, a dodecyl group, a tetradecyl group, a hexadecyl group, an octadecyl group, an icosyl group, a docosyl group, a tetracosyl group, and a hexacosyl group.

Examples of the functional group capable of forming a hydrogen bond with a carboxyl group include a carboxyl group (including a salt thereof), a phosphoric acid group (including a salt thereof), a sulfo group (including a salt thereof), a primary amino group (including a salt thereof), a secondary amino group (including a salt thereof), a tertiary amino group (including a salt thereof), a hydroxyl group, an oxycarbonyl group, a phosphoric acid ester group, a sulfonic acid ester group, an amide group, a urethane group, and a urea group.

In the hydrophobic substance (E), the number of monovalent aliphatic hydrocarbon groups with a carbon number of 8 to 26 is, in view of skin irritation resistance of the absorbent article, preferably from 1 to 5, more preferably from 1 to 4, still more preferably from 1 to 3. In the hydrophobic substance (E), the number of functional groups capable of forming a hydrogen bond with a carboxyl group is, in view of leakage resistance of the absorbent article, preferably from 1 to 5, more preferably from 1 to 4, still more preferably from 1 to 3.

Examples of the hydrophobic substance (E1) having a carboxyl group include a nonanoic acid, a dodecanoic acid, an octadecanoic acid, and a heptacosanoic acid. Examples of the salt thereof include salts with sodium, potassium, zinc, calcium, magnesium and aluminum (hereinafter, simply referred to as Na, K, Zn, Ca, Mg and Al).

Examples of the hydrophobic substance (E2) having a phosphoric acid group include an octylphosphoric acid, an octadecylphosphoric acid, and a hexacosylphosphoric acid. Examples of the salt thereof include salts with Na, K, Zn, Ca, Mg and Al.

Examples of the hydrophobic substance (E3) having a sulfonic acid group include an octylsulfonic acid, an octadecylsulfonic acid, and a hexacosylsulfonic acid. Examples of the salt thereof include salts with Na, K, Zn, Ca, Mg and Al.

Examples of the hydrophobic substance (E4) having a primary amino group include octylamine, octadecylamine, and hexacosylamine. Examples of the salt thereof include salts with hydrochloric acid, carboxylic acid, sulfuric acid and nitric acid.

Examples of the hydrophobic substance (E5) having a secondary amino group include methyloctylamine, methylhexacosylamine, octylhexacosylamine, dihexacosylamine, and methyloctadecylamine. Examples of the salt thereof include salts with hydrochloric acid, carboxylic acid, sulfuric acid and nitric acid.

Examples of the hydrophobic substance (E6) having a tertiary amino group include dimethyloctylamine, dimethylhexacosylamine, methyloctylhexacosylamine, and methyldihexacosylamine. Examples of the salt thereof include salts with hydrochloric acid, carboxylic acid, sulfuric acid and nitric acid.

Examples of the hydrophobic substance (E7) having a hydroxyl group include octyl alcohol, octadecyl alcohol, and hexacosyl alcohol.

Examples of the hydrophobic substance (E8) having an oxycarbonyl group include an esterification product of a long-chain fatty acid with a carbon number of 8 to 26 and an alcohol with a carbon number of 1 to 26 having at least one hydroxyl group, and an esterification product of a long-chain aliphatic alcohol with a carbon number of 8 to 26 and a carboxylic acid having a hydrocarbon group with a carbon number of 1 to 7 and having at least one carboxyl group.

Examples of the esterification product of a long-chain fatty acid with a carbon number of 8 to 26 and an alcohol with a carbon number of 1 to 26 having at least one hydroxyl group include methyl nonanoate, methyl heptacosanoate, hexacosyl nonanoate, hexacosyl heptacosanoate, octyl octadecanoate, glycerin monononanoate, glycerin monooctadecanoate, glycerin monoheptacosanoate, pentaerythritol monononanoate, pentaerythritol monooctadecanoate, pentaerythritol monoheptacosanoate, sorbitol monononanoate, sorbitol monooctadecanoate, sorbitol monoheptacosanoate, sucrose monononanoate, sucrose dinonanoate, sucrose trinonanoate, sucrose monooctadecanoate, sucrose dioctadecanoate, sucrose trioctadecanoate, sucrose monoheptacosanoate, sucrose diheptacosanoate, sucrose triheptacosanoate, and beef tallow.

Examples of the esterification product of a long-chain aliphatic alcohol with a carbon number of 8 to 26 and a carboxylic acid with a carbon number of 1 to 8 having at least one carboxyl group include octyl nonanoate, octyl acetate, octyl octylate, hexacosyl acetate, and hexacosyl octylate.

The hydrophobic substance (E9) having a phosphoric acid ester group includes a dehydrated condensate of a long-chain aliphatic alcohol with a carbon number of 8 to 26 and a phosphoric acid, and a salt thereof. Examples thereof include octyl phosphate, octadecyl phosphate, and hexacosyl phosphate. Examples of the salt thereof include salts with Na and K. The phosphoric acid ester includes diester and triester as well as monoester.

The hydrophobic substance (E10) having a sulfuric acid ester group includes a dehydrated condensate of a long-chain aliphatic alcohol with a carbon number of 8 to 26 and a sulfuric acid, and a salt thereof. Examples thereof include octyl sulfate, octadecyl sulfate, and hexacosyl sulfate. Examples of the salt thereof include salts with Na and K. The sulfuric acid ester includes diester as well as monoester.

The hydrophobic substance (E11) having an amide group includes an amidation product of a long-chain aliphatic primary amine with a carbon number of 8 to 26 and a carboxylic acid having a hydrocarbon group with a carbon number of 1 to 26, an amidation product of ammonia or a primary amine with a carbon number of 1 to 7 and a long-chain fatty acid with a carbon number of 8 to 26, an amidation product of a long-chain aliphatic secondary amine having at least one aliphatic chain with a carbon number of 8 to 26 and a carboxylic acid with a carbon number of 1 to 26, and an amidation product of a secondary amine having two aliphatic hydrocarbon groups with a carbon number of 1 to 7 and a long-chain fatty acid with a carbon number of 8 to 26.

The amidation product of a long-chain aliphatic primary amine with a carbon number of 8 to 26 and a carboxylic acid having a hydrocarbon group with a carbon number of 1 to 26 includes those obtained by reacting a primary amine and a carboxylic acid at 1:1 and at 1:2. Examples of those obtained by the reaction at 1:1 include acetic acid N-octylamide, acetic acid N-hexacosylamide, heptacosanoic acid N-octylamide, and heptacosanoic acid N-hexacosylamide. Examples of those obtained by the reaction at 1:2 include diacetic acid N-octylamide, diacetic acid N-hexacosylamide, diheptacosanoic acid N-octylamide, and diheptacosanoic acid N-hexacosylamide. In the case of reacting a primary amine and a carboxylic acid at 1:2, the carboxylic acids used may be the same or different.

The amidation products of ammonia or a primary amine with a carbon number of 1 to 7 and a long-chain fatty acid with a carbon number of 8 to 26 are classified into those obtained by the reaction of ammonia or a primary amine with a carboxylic acid at 1:1 and those obtained by the reaction at 1:2. Examples those obtained by the reaction at 1:1 include nonanoic acid amide, nonanoic acid methylamide, nonanoic acid N-heptylamide, heptacosanoic acid amide, heptacosanoic acid N-methylamide, heptacosanoic acid N-heptylamide, and heptacosanoic acid N-hexacosylamide. Examples of those obtained by the reaction at 1:2 include dinonanoic acid amide, dinonanoic acid N-ethylamide, dinonanoic acid N-heptylamide, diheptacosanoic acid amide, diheptacosanoic acid N-methylamide, diheptacosanoic acid N-heptylamide, and diheptacosanoic acid N-hexacosylamide. In the case of reacting ammonia or a primary amine with a carboxylic acid at 1:2, the carboxylic acids used may be the same or different.

Examples of the amidation product of a long-chain aliphatic secondary amine having at least one aliphatic chain with a carbon number of 8 to 26 and a carboxylic acid with a carbon number of 1 to 26 include acetic acid N-methyloctylamide, acetic acid N-methylhexacosylamide, acetic acid N-octylhexacosylamide, acetic acid N-dihexacosylamide, heptacosanoic acid N-methyloctylamide, heptacosanoic acid N-methylhexacosylamide, heptacosanoic acid N-octylhexacosylamide, and heptacosanoic acid N-dihexacosylamide.

Examples of the amidation product of a secondary amine having two aliphatic hydrocarbon groups with a carbon number of 1 to 7 and a long-chain fatty acid with a carbon number of 8 to 26 include nonanoic acid N-dimethylamide, nonanoic acid N-methylheptylamide, nonanoic acid N-diheptylamide, heptacosanoic acid N-dimethylamide, heptacosanoic acid N-methylheptylamide, and heptacosanoic acid N-diheptylamide.

The hydrophobic substance (E12) having a urethane group includes a reaction product of a long-chain aliphatic alcohol with a carbon number of 8 to 26 and a compound having at least one isocyanate group. Examples of the long-chain aliphatic alcohol include octyl alcohol, octadecyl alcohol, and hexacosyl alcohol, and examples of the compound having at least one isocyanate group include methyl isocyanate, dodecyl isocyanate, hexacosyl isocyanate, methylene diisocyanate, and hexamethylene diisocyanate.

The hydrophobic substance (E13) having a urea group includes a reaction product of a primary or secondary amine having a hydrocarbon group with a carbon number of 8 to 26 and a compound having at least one isocyanate group. Examples of the primary amine include octylamine, octadecylamine, and hexacosylamine. Examples of the secondary amine include methyloctylamine, methylhexacosylamine, octylhexacosylamine, and dihexacosylamine. Examples of the compound having at least one isocyanate group include methyl isocyanate, dodecyl isocyanate, hexacosyl isocyanate, methylene diisocyanate, and hexamethylene diisocyanate.

Among these hydrophobic substances (E), in view of leakage resistance of the absorbent article, a compound having, as the functional group capable of forming a hydrogen bond with a carboxyl group, at least one member selected from the group consisting of a carboxyl group (including a salt thereof), a phosphoric acid group (including a salt thereof), a sulfo group (including a salt thereof), a primary amino group (including a salt thereof), a secondary amino group (including a salt thereof), a tertiary amino group (including a salt thereof), a hydroxyl group, an oxycarbonyl group, a phosphoric acid ester group, a sulfonic acid ester group and an amide group is preferred, and a compound having at least one member selected from the group consisting of a carboxyl group (including a salt thereof), a primary amino group (including a salt thereof), a secondary amino group (including a salt thereof), a tertiary amino group (including a salt thereof), a hydroxyl group, an oxycarbonyl group and an amide group is more preferred. As for the hydrophobic substance (E), one may be used alone, or two or more may be used in combination.

As described above, the hydrophobic substance (E) is present in the following specific amount in the inside of the absorbent resin particle (P-1) and the absorbent composition particle (P-2) and present in the following specific amount on the surface of (P-1) and (P-2).

In view of skin irritation resistance and leakage resistance in use for an absorbent article, the content of the hydrophobic substance (E) in the inside of (P-1) and (P-2) is preferably from 0.01 to 10.0 wt%, more preferably from 0.01 to 5.0 wt%, still more preferably from 0.05 to 2.0 wt%, and most preferably from 0.1 to 1.0 wt%, based on the weight of (P-1) or (P-2), respectively.

In view of skin irritation resistance and leakage resistance in use for an absorbent article, the content of the hydrophobic substance (E) present on the surface of (P-1) and (P-2) is from 0.001 to 1.0 wt%, preferably from 0.005 to 0.5 wt%, more preferably from 0.01 to 0.3 wt%, still more preferably from 0.01 to 0.1 wt%, based on the weight of (P-1) or (P-2), respectively.

The content of the hydrophobic substance (E) present on the surface is measured by the following method. Also, the content of the hydrophobic substance (E) present in the inside is calculated from the amount obtained by subtracting the amount of the hydrophobic substance (E) present on the surface from the amount of the hydrophobic substance (E) used for the production.

<Measurement Method of Content of Hydrophobic Substance (E) >

1 Part by weight of the absorbent resin particle (P-1) or absorbent composition particle (P-2) and 1,000 parts by weight of an organic solvent (an organic solvent capable of dissolving at least 0.01 parts by weight of the hydrophobic substance per 100 parts by weight of the organic solvent at 25 to 110°C; here, the temperature allowing for this dissolution is referred to as dissolution temperature) are added to a glass-made beaker and left standing at the dissolution temperature for 24 hours to obtain an extraction solution of the hydrophobic substance. The extraction solution is filtrated using a filter paper and collected into a glass-made beaker weighed in advance, and after evaporating the solvent, the beaker is weighed. The numerical value obtained by subtracting the weight of the beaker weighed in advance from the weight after evaporation of the filtration solution is multiplied by 100. Using the sample after extraction remaining on the filter paper, the same operation is repeated twice, and the total amount of the evaporation-to-dryness matters obtained by three-time extraction is taken as the content (wt%) of the hydrophobic substance present on the surface.

In the measurement method of swelled volume per 1 g of the absorbent resin particle of the present invention for physiological saline, the ratio (t2/t1) between the time (t1) until the swelled volume reaches 5 ml and the time (t2) until the swelled volume reaches 40 ml is preferably from 5 to 20, more preferably from 5 to 15, and most preferably from 5 to 10. Also, t1 is preferably from 20 to 60 seconds, more preferably from 20 to 50 seconds, and most preferably from 30 to 40 seconds. Within this range, the skin irritation resistance of an absorbent article is further improved.

The swelled volume is measured by the following method.

<Measuring Apparatus>

The measurement method of swelled volume is a measurement method performed in a room at 25±2°C and a humidity of 50±10% typically by using the apparatus shown in Fig. 1. In this connection, the temperature of the physiological saline used is 25±2°C. The apparatus shown in Fig. 1 is composed of an acryl-made bottomed cylinder 1 and an acryl-made disk 2. Incidentally, numerical values relevant to the apparatus in the following description are an example, and the present invention is not limited to these numerical values. The numerical values relevant to the measurement method are also an example.

The bottomed cylinder 1 is a bottomed cylinder having a bottom at one opening of a cylinder with an inner diameter of 81 mm and a length of 35 mm and being opened at another side. The acryl-made disk 2 is a disk with an outer diameter of 80.5 mm and a thickness of 12 mm. The disk 2 having a circular concave with a diameter of 70.5 mm and a depth of 4 mm at a position where the center of the disk is coincident with the center of the circle. Also, the disk 2 has, as a handgrip, a column with a length of 13 mm and an outer diameter of 15 mm in the circular concave portion, at a position where the center of the disk 2 is coincident with the center of bottom face of the column. Furthermore, in the disk 2, 64 holes with a diameter of 2 mm are perforated in a radial pattern. The holes of the disk 2 are described below. The holes are perforated such that 5 holes with a diameter of 2 mm are present on each of lines dividing the disk into equal eight portions and are located at equal intervals of 5 mm between a position of 10 mm and a position of 30 mm from the center of the disk (40 holes in total). In addition, 3 holes with a diameter of 2 mm are present on each of lines dividing the disk into equal eight portions and being tilted at 22.5° from the above-described equally dividing lines and are located at equal intervals of 5 mm between a position of 20 mm and a position of 30 mm from the center of the disk (24 holes in total). The weight of the disk 2 is 60±5 g.

<Measurement Procedure of Swelled Volume>

Into the bottom plate-attached cylinder 1 erected vertically, 2.50 g of a measurement sample sieved to a particle diameter of 150 to 850 $\mu$m (water content ratio: 8.0% or less) is weighed and charged to lie at an almost uniform thickness on the bottom of the bottom plate-attached cylinder 1. The disk 2 is placed thereon with the columnar handgrip up, and the distance from the bottom face of the cylinder to the top face of the handgrip of the disk is measured using a thickness meter (for example, Digimatic Indicator ID-F150, manufactured by Mitutoyo Co.). At this time, the pressure imposed on the measurement sample due to weight of the measuring rod (140±10 g) of the Digimatic indicator and weight of the disk 2 with the handgrip is 3.9±0.3 g/cm$^2$. Then, the thickness indication of the Digimatic indicator is set zero. Subsequently, 120 ml of physiological saline is charged into the bottomed cylinder 1 within 2 seconds. The time of the start of charging is taken as zero, and the distance H (cm) for which the disk 2 is elevated with the passage of time from the start of charging is recorded as continuous data. The swelled volume (ml) per 1 g of the measurement sample is calculated according to the following formula, whereby data of change in swelled volume with respect to the time are obtained. From the data, the time (t1) until the swelled volume reaches 5 ml and the time (t2) until the swelled volume reaches 40 ml are determined. The measurement is performed five times, and the average value thereof is used as the measured value.

[Math. 1]

$$\text{Swelled volume (ml/g)} = \text{bottom area (cm}^2\text{) within bottom plate-attached cylinder} \times H \text{ (cm)/weight (g) of measurement sample}$$

Examples of the method for incorporating the hydrophobic substance (E) into the absorbent resin (C) particle and the absorbent composition (D) particle to produce the absorbent resin particle (P-1) and the absorbent composition particle (P-2) include (1) a method of mixing/kneading the hydrophobic substance (E) and a hydrous gel of (C) or (D) at preferably from 20 to 100°C, more preferably from 40 to 90°C, still more preferably from 50 to 80°C, and then forming the mixture into particles, and (2) a method of producing (B1) and/or (B2) in the presence of the hydrophobic substance (E) and (A1) and/or (A2), and then forming the product into particles.

In the production process of the absorbent resin (C) and the absorbent composition (D), the timing of binding or mixing the (A1) and/or (A2) and the (B1) and/or (B2) is described below by referring to the following general steps [1] to [4] relevant to (B1) and/or (B2):

[1] a step of subjecting a (meth)acrylic acid monomer and/or its salt obtained by neutralization with a neutralizer to radial polymerization to produce (B1) and/or (B2),
[2] a step of neutralizing the (B1) and/or (B2),
[3] a step of drying the (B1) and/or (B2), and
[4] a step of surface-crosslinking the (B1) and/or (B2).

In step [1], (B1) and/or (B2) is produced in the presence of the (A1) and/or (A2), whereby an absorbent resin (C) in which (B1) and/or (B2) are grafted to (A1) and/or (A2) is obtained.

Between step [1] and step [2] or between step [2] and step [3], a step of binding the (A1) and/or (A2) to the (B1) and/or (B2) with a binder is provided, whereby an absorbent resin (C) is obtained.

In step (3), a binding reaction of (A1) and/or (A2) to (B1) and/or (B2) is performed in the course of drying by allowing (A1) and/or (A2) and a binder to be present together, whereby the absorbent resin (C) is obtained.

In the absorbent composition (D), mixing of the (A1) and/or (A2), the (B1) and/or (B2), and the absorbent resin (C) that is used, if desired, may be performed at arbitrary timing as long as it is after the completion of step [1]. However, when mixed after the completion of step [3], since (B1) and/or (B2) are a solid, the absorbent composition (D) is obtained in the form of a powder blend. Also, (A1) and/or (A2) used after the completion of step [3] may be subjected to a surface crosslinking treatment. Furthermore, when a mixture of the (A1) and/or (A2) and the (B1) and/or (B2) is surface-crosslinked in step [4], the absorbent composition (D) can be obtained as a composition having the absorbent resin (C) only on the surface.

The absorbent resin, absorbent resin particle, absorbent composition and absorbent resin particle of the present invention can be individually or in combination used together with at least one member selected from the group consisting of a fiber, a nonwoven fabric and a woven fabric to produce an absorber.

Examples of the fiber, nonwoven fabric and woven fabric include fibrous materials [the raw material (e.g., softwood, hardwood), the production method {e.g., chemical pulp, semichemical pulp, chemithermomechanical pulp (CTMP)}, the bleaching method and the like are not particularly limited] conventionally used for an absorbent article, such as various fluff pulps and cotton-like pulps. In addition to these fibrous materials, a non-water-swellable synthetic fiber may be also used by itself or in combination with the above-described fluff pulp, cotton-like pulp or the like. Examples of the synthetic fiber include a polyolefin-based fiber (such as polyethylene-based fiber and polypropylene-based fiber), a polyester-based fiber (such as polyethylene terephthalate fiber), a polyolefin/polyester composite fiber, a polyamide-based fiber, and a polyacrylonitrile-based fiber. The structure, production method and the like of the absorber are the same as those conventionally known (for example, JP-A-2003-225565).

This absorber is suitably used for an absorbent article [such as disposable diaper, sanitary napkin, paper towel, pad (e.g., incontinence pad, surgical underpad) and pet sheet]. The production method and the like of the absorbent article are the same as those conventionally known (for example, JP-A-2003-225565).

In the case of using the absorbent resin, absorbent resin particle, absorbent composition and absorbent composition particle of the present invention together with at least one member selected from the group consisting of a fiber, a nonwoven fabric and a woven fabric to produce an absorber, the weight ratio thereof to the fiber or the like (weight of the absorbent resin particle or the like/weight of fiber or the like) is preferably from 40/60 to 70/30, more preferably from 50/50 to 60/40.

EXAMPLES

The present invention is further described below by referring to Examples and Comparative Examples, but the present invention is not limited thereto. In the following, "parts" indicates "parts by weight".

<Production Example 1>

Production of Hydrous Gel of Crosslinked Polyacrylic Acid:

155 Parts (2.15 molar parts) of acrylic acid (produced by Mitsubishi Chemical Corporation, purity: 100%), 0.6225 parts (0.0024 molar parts) of pentaerythritol triallyl ether (produced by Daiso Co., Ltd.) as an internal crosslinking agent, and 340.27 parts of deionized water were kept at 3°C with stirring/mixing. After flowing nitrogen into the mixture to make a dissolved oxygen concentration of 1 ppm or less, 0.62 parts of an aqueous 1% hydrogen peroxide solution, 1.1625 parts of an aqueous 2% ascorbic acid solution, 2.325 parts of an aqueous 2% 2,2'-azobis[2-methyl-N-(2-hydroxyethyl)-propionamide] solution, and 1.9 parts of Surfactant (A-1) (hexadecanoic acid ethylene oxide 2-mol adduct) were added/mixed to initiate polymerization. After the temperature of the mixture reached 90°C, polymerization was allowed to proceed at $90\pm2$°C for about 5 hours, whereby 502.27 parts by weight of Hydrous Gel (B2-1) of crosslinked polyacrylic acid was obtained.

<Production Example 2>

Production of Aqueous Solution of Linear Polyacrylic Acid:

501.65 Parts by weight of Aqueous Solution (B1-1) of linear polyacrylic acid was obtained in the same manner as in Production Example 1 except for not adding pentaerythritol triallyl ether as an internal crosslinking agent.

<Production Example 3>

Production of Aqueous Solution (A1-1) of Polysaccharide oxide:

5 Parts of starch (MS-3800, produced by Nihon Shokuhin Kako Co., Ltd.) and 100 parts of ion-exchanged water were added and stirred. 32.6 Parts of potassium permanganate was dissolved in 500 parts of ion-exchanged water, and the resulting solution was added over 6 hours. During the addition, pH was kept at 12 by adding an aqueous 2 N sodium hydroxide (NaOH) solution. After the completion of the addition of potassium permanganate, stirring was further continued for 2 hours, and the unreacted potassium permanganate was reduced with sodium sulfite. Manganese dioxide as a by-product was separated by filtration, and the filtrate was purified by performing diffusion dialysis with ion-exchanged water for 5 days and then concentrated to obtain Aqueous Solution (A1-1) of 30 wt% polysaccharide oxide. Mw of the obtained polysaccharide oxide was 6,000, and the acid value was 700 mgKOH/g.

<Production Example 4>

Production of Aqueous Solution (A1-2) of Polysaccharide oxide:

Aqueous Solution (A1-2) of 30 wt% polysaccharide oxide was obtained in the same manner as in Production Example 3 except for using corn starch (produced by Wako Pure Chemical Industries, Ltd.) in place of starch and changing the charge amount of potassium permanganate to 28 parts and the addition time to 2 hours. Mw of the obtained polysaccharide oxide was 900,000, and the acid value was 536 mgKOH/g.

<Production Example 5>

Production of Aqueous Solution (A1-3) of Polysaccharide oxide:

Aqueous Solution (A1-3) of 30 wt% polysaccharide oxide was obtained in the same manner as in Production Example 4 except for changing the charge amount of potassium permanganate to 40 parts and the addition time to 8 hours. Mw of the obtained polysaccharide oxide was 450,000, and the acid value was 800 mgKOH/g.

<Production Example 6>

Production of Aqueous Solution (A1-4) of Polysaccharide oxide:

Aqueous Solution (A1-4) of 30 wt% polysaccharide oxide was obtained in the same manner as in Production Example 3 except for using Kiprogum (M-800A, produced by Nippon Starch Chemical Co., Ltd.) in place of starch. Mw of the obtained polysaccharide oxide was 85,000, and the acid value was 750 mgKOH/g.

<Production Example 7>

Production of Aqueous Solution (A1-5) of Polysaccharide oxide:

While stirring 31.9 parts of an aqueous 12 wt% sodium hydroxide solution, 5 parts of cellulose (KC FLOCK W-400G, produced by Nippon Paper Chemicals Co., Ltd.) was added thereto and uniformly mixed at 25°C for 90 minutes to mercerize the cellulose. 40.0 Parts of potassium permanganate was dissolved in 613 parts of ion-exchanged water, and the resulting solution was added over 10 hours. During the addition, pH was kept at 12 by adding an aqueous 2 mol/L sodium hydroxide solution. The subsequent procedure was performed in the same manner as in Production Example 3 to obtain Aqueous Solution (A1-5) of 30 wt% polysaccharide oxide. Mw of the obtained polysaccharide oxide was 320,000, and the acid value was 610 mgKOH/g.

<Production Example 8>

Production of Aqueous Solution (A1-6) of Polysaccharide oxide:

Aqueous Solution (A1-6) of 30 wt% polysaccharide oxide was obtained in the same manner as in Production Example 3 except for using 6.3 parts of carboxymethyl cellulose (produced by Aldrich Chemical Co. Inc., substitution degree: 0.7) in place of 5 parts of starch. Mw of the obtained polysaccharide oxide was 300,000, and the acid value was 525 mgKOH/g.

<Production Example 9>

Production of Hydrous Gel (A2-1) of Crosslinked Product of Polysaccharide oxide:

21.6 Parts of an aqueous 48.5 wt% sodium hydroxide solution and 0.5 parts of a 2 wt% water/methanol mixed solution (weight ratio of water/methanol = 70/30) of ethylene glycol diglycidyl ether were added per 100 parts of Aqueous Solution (A1-1) of 30 wt% polysaccharide oxide produced in Production Example 3, and the mixture was stirred at 90°C for 30 minutes to obtain 120.1 parts of Hydrous Gel (A2-1) of crosslinked product of polysaccharide oxide.

<Production Example 10>

Production of Hydrous Gel (A2-2) of Crosslinked Product of Polysaccharide oxide:

116.5 Parts of Hydrous Gel (A2-2) of crosslinked product of polysaccharide oxide was obtained in the same manner as in Production Example 9 except for using Aqueous  Solution (A1-2) of polysaccharide oxide produced in Production Example 4 in place of Aqueous Solution (A1-1) of polysaccharide oxide and changing the charge amount of an aqueous 48.5 wt% sodium hydroxide solution to 16.6 parts.

<Production Example 11>

Production of Hydrous Gel (A2-3) of Crosslinked Product of Polysaccharide oxide:

123.6 Parts of Hydrous Gel (A2-3) of crosslinked product of polysaccharide oxide was obtained in the same manner as in Production Example 9 except for using Aqueous Solution (A1-3) of polysaccharide oxide produced in Production Example 5 in place of Aqueous Solution (A1-1) of polysaccharide oxide and changing the charge amount of an aqueous 48.5 wt% sodium hydroxide solution to 24.7 parts.

<Production Example 12>

Production of Hydrous Gel (A2-4) of Crosslinked Product of Polysaccharide oxide:

122.3 Parts of Hydrous Gel (A2-4) of crosslinked product of polysaccharide oxide was obtained in the same manner as in Production Example 9 except for using Aqueous Solution (A1-4) of polysaccharide oxide produced in Production Example 6 in place of Aqueous Solution (A1-1) of polysaccharide oxide and changing the charge amount of an aqueous 48.5 wt% sodium hydroxide solution to 23.2 parts.

<Production Example 13>

Production of Hydrous Gel (A2-5) of Crosslinked Product of Polysaccharide oxide:

118.1 Parts of Hydrous Gel (A2-5) of crosslinked  product of polysaccharide oxide was obtained in the same manner as in Production Example 9 except for using Aqueous Solution (A1-5) of polysaccharide oxide produced in Production Example 7 in place of Aqueous Solution (A1-1) of polysaccharide oxide and changing the charge amount of an aqueous 48.5 wt% sodium hydroxide solution to 18.9 parts.

<Production Example 14>

Production of Hydrous Gel (A2-6) of Crosslinked Product of Polysaccharide oxide:

116.0 Parts of Hydrous Gel (A2-6) of crosslinked product of polysaccharide oxide was obtained in the same manner as in Production Example 9 except for using Aqueous Solution (A1-6) of polysaccharide oxide produced in Production Example 8 in place of Aqueous Solution (A1-1) of polysaccharide oxide and changing the charge amount of an aqueous 48.5 wt% sodium hydroxide solution to 16.2 parts.

<Example 1>

Production of Hydrous Gel (G1) of Graft Polymer:

93 Parts (1.29 molar parts) of acrylic acid (produced by Mitsubishi Chemical Corporation, purity: 100%), 0.3735 parts (0.0014 molar parts) of pentaerythritol triallyl ether (produced by Daiso Co., Ltd.) as an internal crosslinking agent, 2.475 parts of a 2 wt% water/methanol mixed solution (weight ratio of water/methanol = 70/30) of ethylene glycol diglycidyl ether, and 340.27 parts of deionized water having previously dissolved therein 206.7 parts of Aqueous Solution (A1-1) of polysaccharide oxide produced in Production Example 3 were kept at 3°C with stirring/mixing. After flowing nitrogen into the mixture to make a dissolved oxygen concentration of 1 ppm or less, 0.372 parts of an aqueous 1% hydrogen peroxide solution, 0.698 parts of an aqueous 2% ascorbic acid solution, and 1.395 parts of an aqueous 2% 2,2'-azobis [2-methyl-N-(2-hydroxyethyl)-propionamide] solution were added/mixed to initiate polymerization. After the temperature of the mixture reached 90°C, polymerization was allowed to proceed at $90 \pm 2$°C for about 5 hours, whereby 501.01 parts of Hydrous Gel (G1) of graft polymer was obtained.

<Example 2>

Production of Hydrous Gel (G2) of Graft Polymer:

Hydrous Gel (G2) of graft polymer was obtained in the same manner as in Example 1 except for using Aqueous Solution (A1-2) of polysaccharide oxide in place of Aqueous Solution (A1-1) of polysaccharide oxide.

<Example 3>

Production of Hydrous Gel (G3) of Graft Polymer:

Hydrous Gel (G3) of graft polymer was obtained in the same manner as in Example 1 except for changing the charge amount of acrylic acid to 108.5 parts and using 155.5 parts of Aqueous Solution (A1-3) of polysaccharide oxide in place of 206.7 parts of Aqueous Solution (A1-1) of polysaccharide oxide.

<Example 4>

Production of Hydrous Gel (G4) of Graft Polymer:

Hydrous Gel (G4) of graft polymer was obtained in the same manner as in Example 1 except for using Aqueous Solution (A1-3) of polysaccharide oxide in place of Aqueous Solution (A1-1) of polysaccharide oxide.

<Example 5>

Production of Hydrous Gel (G5) of Graft Polymer:

Hydrous Gel (G5) of graft polymer was obtained in the same manner as in Example 1 except for changing the charge amount of acrylic acid to 31 parts and using 413.3 parts of Aqueous Solution (A1-3) of polysaccharide oxide in place of 206.7 parts of Aqueous Solution (A1-1) of polysaccharide oxide.

<Example 6>

Production of Hydrous Gel (G6) of Graft Polymer:

Hydrous Gel (G6) of graft polymer was obtained in the same manner as in Example 1 except for using Aqueous Solution (A1-4) of polysaccharide oxide in place of Aqueous Solution (A1-1) of polysaccharide oxide.

<Example 7>

Production of Hydrous Gel (G7) of Graft Polymer:

Hydrous Gel (G7) of graft polymer was obtained in the same manner as in Example 1 except for using Aqueous Solution (A1-5) of polysaccharide oxide in place of Aqueous Solution (A1-1) of polysaccharide oxide.

<Example 8>

Production of Hydrous Gel (G8) of Graft Polymer:

Hydrous Gel (G8) of graft polymer was obtained in the same manner as in Example 1 except for changing the charge amount of acrylic acid to 108.5 parts and using 155.5 parts of Aqueous Solution (A1-6) of polysaccharide oxide in place  of 206.7 parts of Aqueous Solution (A1-1) of polysaccharide oxide.

<Example 9>

Production of Hydrous Gel (G9) of Graft Polymer:

Hydrous Gel (G9) of graft polymer was obtained in the same manner as in Example 1 except for using Aqueous Solution (A1-6) of polysaccharide oxide in place of Aqueous Solution (A1-1) of polysaccharide oxide.

<Example 10>

Production of Hydrous Gel (G10) of Graft Polymer:

Hydrous Gel (G10) of graft polymer was obtained in the same manner as in Example 1 except for changing the charge amount of acrylic acid to 31 parts and using 413.3 parts of Aqueous Solution (A1-6) of polysaccharide oxide in place of 206.7 parts of Aqueous Solution (A1-1) of polysaccharide oxide.

<Example 11>

Production of Hydrous Gel (G11) of Graft Polymer:

Hydrous Gel (G11) of graft polymer was obtained in the same manner as in Example 1 except for not adding pentaerythritol triallyl ether (produced by Daiso Co., Ltd.) as an internal crosslinking agent.

<Example 12>

Production of Hydrous Gel (G12) of Graft Polymer:

Hydrous Gel (G12) of graft polymer was obtained in the same  manner as in Example 11 except for using Aqueous Solution (A1-2) of polysaccharide oxide in place of Aqueous  Solution (A1-1) of polysaccharide oxide.

<Example 13>

Production of Hydrous Gel (G13) of Graft Polymer:

Hydrous Gel (G9) of graft polymer was obtained in the same manner as in Example 11 except for changing the charge amount of acrylic acid to 108.5 parts and using 155.5 parts of Aqueous Solution (A1-3) of polysaccharide oxide in place of 206.7 parts of Aqueous Solution (A1-1) of polysaccharide oxide.

<Example 14>

Production of Hydrous Gel (G14) of Graft Polymer:

Hydrous Gel (G14) of graft polymer was obtained in the same manner as in Example 11 except for using Aqueous Solution (A1-3) of polysaccharide oxide in place of Aqueous Solution (A1-1) of polysaccharide oxide.

<Example 15>

Production of Hydrous Gel (G15) of Graft Polymer:

Hydrous Gel (G15) of graft polymer was obtained in the same manner as in Example 11 except for changing the charge amount of acrylic acid to 31 parts and using 413.3 parts of Aqueous Solution (A1-3) of polysaccharide oxide in place of 206.7 parts of Aqueous Solution (A1-1) of polysaccharide oxide.

<Example 16>

Production of Hydrous Gel (G16) of Graft Polymer:

Hydrous Gel (G16) of graft polymer was obtained in the same manner as in Example 11 except for using Aqueous  Solution (A1-4) of polysaccharide oxide in place of Aqueous Solution (A1-1) of polysaccharide oxide.

<Example 17>

Production of Hydrous Gel (G17) of Graft Polymer:

Hydrous Gel (G17) of graft polymer was obtained in the same manner as in Example 11 except for using Aqueous Solution (A1-5) of polysaccharide oxide in place of Aqueous Solution (A1-1) of polysaccharide oxide.

<Example 18>

Production of Hydrous Gel (G18) of Graft Polymer:

Hydrous Gel (G18) of graft polymer was obtained in the same manner as in Example 11 except for changing the charge amount of acrylic acid to 108.5 parts and using 155.5 parts of Aqueous Solution (A1-6) of polysaccharide oxide in place of 206.7 parts of Aqueous Solution (A1-1) of polysaccharide oxide.

<Example 19>

Production of Hydrous Gel (G19) of Graft Polymer:

Hydrous Gel (G19) of graft polymer was obtained in the same manner as in Example 11 except for using Aqueous Solution (A1-6) of polysaccharide oxide in place of Aqueous Solution (A1-1) of polysaccharide oxide.

<Example 20>

Production of Hydrous Gel (G20) of Graft Polymer:

Hydrous Gel (G20) of graft polymer was obtained in the same manner as in Example 11 except for changing the charge amount of acrylic acid to 31 parts and using 413.3  parts of Aqueous Solution (A1-6) of polysaccharide oxide in place of 206.7 parts of Aqueous Solution (A1-1) of polysaccharide oxide.

<Example 21>

Production of Hydrous Gel (G21) of Graft Polymer:

93 Parts (1.29 molar parts) of acrylic acid (produced by Mitsubishi Chemical Corporation, purity: 100%), 0.3735 parts (0.0014 molar parts) of pentaerythritol triallyl ether (produced by Daiso Co., Ltd.) as an internal crosslinking agent, and 340.27 parts of deionized water having previously dissolved therein 206.7 parts of Aqueous Solution (A1-1) of polysaccharide oxide produced in Production Example 3 were kept at 3°C with stirring/mixing. After flowing nitrogen into the mixture to make a dissolved oxygen concentration of 1 ppm or less, 0.372 parts of an aqueous 1% hydrogen peroxide solution, 0.698 parts of an aqueous 2% ascorbic acid solution, and 1.395 parts of an aqueous 2% 2,2'-azobis[2-methyl-N-(2-hydroxyethyl)-propionamide] solution were added/mixed to initiate polymerization. After the temperature of the mixture reached 90°C, polymerization was allowed to proceed at 90±2°C for about 5 hours, whereby 501.01 parts of Hydrous Gel (G21) of graft polymer was obtained.

<Example 22>

Production of Hydrous Gel (G22) of Graft Polymer:

Hydrous Gel (G22) of graft polymer was obtained in the same manner as in Example 21 except for using Aqueous Solution (A1-2) of polysaccharide oxide in place of Aqueous Solution (A1-1) of polysaccharide oxide.

<Example 23>

Production of Hydrous Gel (G23) of Graft Polymer:

Hydrous Gel (G23) of graft polymer was obtained in the same manner as in Example 21 except for changing the charge amount of acrylic acid to 108.5 parts and using 155.5 parts of Aqueous Solution (A1-3) of polysaccharide oxide in place of 206.7 parts of Aqueous Solution (A1-1) of polysaccharide oxide.

<Example 24>

Production of Hydrous Gel (G24) of Graft Polymer:

Hydrous Gel (G24) of graft polymer was obtained in the same manner as in Example 21 except for using Aqueous Solution (A1-3) of polysaccharide oxide in place of Aqueous Solution (A1-1) of polysaccharide oxide.

<Example 25>

Production of Hydrous Gel (G25) of Graft Polymer:

Hydrous Gel (G25) of graft polymer was obtained in the same manner as in Example 21 except for changing the charge amount of acrylic acid to 31 parts and using 413.3 parts of Aqueous Solution (A1-3) of polysaccharide oxide in place of 206.7 parts of Aqueous Solution (A1-1) of polysaccharide oxide.

<Example 26>

Production of Hydrous Gel (G26) of Graft Polymer:

Hydrous Gel (G26) of graft polymer was obtained in the same manner as in Example 21 except for using Aqueous Solution (A1-4) of polysaccharide oxide in place of Aqueous Solution (A1-1) of polysaccharide oxide.

<Example 27>

Production of Hydrous Gel (G27) of Graft Polymer:

Hydrous Gel (G27) of graft polymer was obtained in the same manner as in Example 21 except for using Aqueous Solution (A1-5) of polysaccharide oxide in place of Aqueous Solution (A1-1) of polysaccharide oxide.

<Example 28>

Production of Hydrous Gel (G28) of Graft Polymer:

Hydrous Gel (G28) of graft polymer was obtained in the same manner as in Example 21 except for changing the charge amount of acrylic acid to 108.5 parts and using 155.5 parts of Aqueous Solution (A1-6) of polysaccharide oxide in place of 206.7 parts of Aqueous Solution (A1-1) of polysaccharide oxide.

<Example 29>

Production of Hydrous Gel (G29) of Graft Polymer:

Hydrous Gel (G29) of graft polymer was obtained in the same manner as in Example 21 except for using Aqueous Solution (A1-6) of polysaccharide oxide in place of Aqueous Solution (A1-1) of polysaccharide oxide.

<Example 30>

Production of Hydrous Gel (G30) of Graft Polymer:

Hydrous Gel (G30) of graft polymer was obtained in the same manner as in Example 21 except for changing the charge amount of acrylic acid to 31 parts and using 413.3 parts of Aqueous Solution (A1-6) of polysaccharide oxide in  place of 206.7 parts of Aqueous Solution (A1-1) of polysaccharide oxide.

<Example 31>

Production of Hydrous Gel (G31) of Graft Polymer:

Hydrous Gel (G31) of graft polymer was obtained in the same manner as in Example 21 except for not adding pentaerythritol triallyl ether (produced by Daiso Co., Ltd.).

<Example 32>

Production of Hydrous Gel (G32) of Graft Polymer:

Hydrous Gel (G32) of graft polymer was obtained in the same manner as in Example 31 except for using Aqueous Solution (A1-2) of polysaccharide oxide in place of Aqueous Solution (A1-1) of polysaccharide oxide.

<Example 33>

Production of Hydrous Gel (G33) of Graft Polymer:

Hydrous Gel (G33) of graft polymer was obtained in the same manner as in Example 31 except for changing the charge amount of acrylic acid to 108.5 parts and using 155.5 parts of Aqueous Solution (A1-3) of polysaccharide oxide in place of 206.7 parts of Aqueous Solution (A1-1) of polysaccharide oxide.

<Example 34>

Production of Hydrous Gel (G34) of Graft Polymer:

Hydrous Gel (G34) of graft polymer was obtained in the same manner as in Example 31 except for using Aqueous Solution (A1-3) of polysaccharide oxide in place of Aqueous  Solution (A1-1) of polysaccharide oxide.

<Example 35>

Production of Hydrous Gel (G35) of Graft Polymer:

Hydrous Gel (G35) of graft polymer was obtained in the same manner as in Example 31 except for changing the charge amount of acrylic acid to 31 parts and using 413.3 parts of Aqueous Solution (A1-3) of polysaccharide oxide in place of 206.7 parts of Aqueous Solution (A1-1) of polysaccharide oxide.

<Example 36>

Production of Hydrous Gel (G36) of Graft Polymer:

Hydrous Gel (G36) of graft polymer was obtained in the same manner as in Example 31 except for using Aqueous Solution (A1-4) of polysaccharide oxide in place of Aqueous Solution (A1-1) of polysaccharide oxide.

<Example 37>

Production of Hydrous Gel (G37) of Graft Polymer:

Hydrous Gel (G37) of graft polymer was obtained in the same manner as in Example 31 except for using Aqueous Solution (A1-5) of polysaccharide oxide in place of Aqueous Solution (A1-1) of polysaccharide oxide.

<Example 38>

Production of Hydrous Gel (G38) of Graft Polymer:

Hydrous Gel (G38) of graft polymer was obtained in the same manner as in Example 31 except for changing the charge amount of acrylic acid to 108.5 parts and using 155.5 parts of Aqueous Solution (A1-6) of polysaccharide oxide in place of 206.7 parts of Aqueous Solution (A1-1) of polysaccharide oxide.

<Example 39>

Production of Hydrous Gel (G39) of Graft Polymer:

Hydrous Gel (G39) of graft polymer was obtained in the same manner as in Example 31 except for using Aqueous Solution (A1-6) of polysaccharide oxide in place of Aqueous Solution (A1-1) of polysaccharide oxide.

<Example 40>

Production of Hydrous Gel (G40) of Graft Polymer:

Hydrous Gel (G40) of graft polymer was obtained in the same manner as in Example 31 except for changing the charge amount of acrylic acid to 31 parts and using 413.3 parts of Aqueous Solution (A1-6) of polysaccharide oxide in place of 206.7 parts of Aqueous Solution (A1-1) of polysaccharide oxide.

<Examples 41 to 60>

According to the charge formulation in Table 1, an aqueous 48.5 wt% sodium hydroxide solution was added and mixed while mincing a hydrous gel of (B2) a crosslinked product of polyacrylic acid and (A1) an aqueous solution of polysaccharide oxide or a hydrous gel of (A2) a crosslinked product of polysaccharide oxide by a mincer (12VR-400K manufactured by ROYAL Co.) to obtain a minced gel. The minced gel was dried in a through-flow band-type dryer (150°C, wind velocity: 2 m/sec) to obtain a dry form. The dry form was pulverized in a juicer-mixer (OSTERIZER BLENDER, manufactured by Oster Co.) and then adjusted to a particle size of 150 to 710 $\mu$m by using sieves with a sieve-opening of 150 and 710 $\mu$m, whereby dry form particles were obtained. While stirring 100 parts of the dry form particles at a high speed (high-speed stirring turbulizer manufactured by Hosokawa Micron Corporation; rotation speed: 2,000 rpm), a solution of surface crosslinking agent was added and mixed by spraying, and the system was allowed to stand at 150°C for 30 minutes so as to effect surface crosslinking, whereby absorbent compositions of Examples 41 to 60 were obtained. The measurement results of water-retention amount and gel elastic modulus of the obtained absorbent compositions are shown in Table 1. Here, in Table 1, the solution of surface crosslinking agent is a 2 wt% water/methanol (weight ratio = 70/30) mixed solution of ethylene glycol diglycidyl ether.

[Table 1]

| Table 1 | | Example | | | | | | | | | | | | | | | | | | |
|---|---|---|---|---|---|---|---|---|---|---|---|---|---|---|---|---|---|---|---|---|
| | | 41 | 42 | 43 | 44 | 45 | 46 | 47 | 48 | 49 | 50 | 51 | 52 | 53 | 54 | 55 | 56 | 57 | 58 | 59 | 60 |
| Hydrous gel (parts) of (B2) cross-linked product of polyacrylic acid | (B2-1) | 60 | 60 | 70 | 60 | 20 | 60 | 60 | 70 | 60 | 60 | 60 | 70 | 60 | 20 | 60 | 60 | 70 | 60 | 20 | 20 |
| Aqueous solution (parts) of (A1) polysaccharide oxide | (A1-1) | 40 | - | - | - | - | - | - | - | - | - | - | - | - | - | - | - | - | - | - | - |
| | (A1-2) | - | 40 | - | - | - | - | - | - | - | - | - | - | - | - | - | - | - | - | - | - |
| | (A1-3) | - | - | 30 | 40 | 80 | - | - | - | - | - | - | - | - | - | - | - | - | - | - | - |
| | (A1-4) | - | - | - | - | - | 40 | - | - | - | - | - | - | - | - | - | - | - | - | - | - |
| | (A1-5) | - | - | - | - | - | - | 40 | - | - | - | - | - | - | - | - | - | - | - | - | - |
| | (A1-6) | - | - | - | - | - | - | - | 30 | 40 | - | - | - | - | - | - | - | - | - | - | 80 |
| Hydrous gel (parts) of (A2) cross-linked product of polysaccharide oxide | (A2-1) | - | - | - | - | - | - | - | - | - | 40 | - | - | - | - | - | - | - | - | - | - |
| | (A2-2) | - | - | - | - | - | - | - | - | - | - | 40 | - | - | - | - | - | - | - | - | - |
| | (A2-3) | - | - | - | - | - | - | - | - | - | - | - | 30 | 40 | 80 | - | - | - | - | - | - |
| | (A2-4) | - | - | - | - | - | - | - | - | - | - | - | - | - | - | 40 | - | - | - | - | - |
| | (A2-5) | - | - | - | - | - | - | - | - | - | - | - | - | - | - | - | 40 | - | - | - | - |
| | (A2-6) | - | - | - | - | - | - | - | - | - | - | - | - | - | - | - | - | 30 | 40 | 80 | - |

(continued)

EP 2 690 133 A1

| Table 1 | Example | | | | | | | | | | | | | | | | | | | |
|---|---|---|---|---|---|---|---|---|---|---|---|---|---|---|---|---|---|---|---|---|
| | 41 | 42 | 43 | 44 | 45 | 46 | 47 | 48 | 49 | 50 | 51 | 52 | 53 | 54 | 55 | 56 | 57 | 58 | 59 | 60 |
| Aqueous 48.5 wt% NaOH solution (parts) | 23.1 | 21.1 | 24.3 | 24.3 | 24.6 | 23.7 | 22.0 | 21.7 | 20.9 | 17.8 | 23.1 | 23.1 | 24.3 | 24.3 | 24.6 | 23.7 | 22.0 | 21.7 | 20.9 | 17.8 |
| Solution (parts) of surface crosslinking agent | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | 37 | 38 | 36 | 39 | 42 | 39 | 37 | 38 | 36 | 33 | 37 | 38 | 37 | 39 | 40 | 35 | 39 | 40 | 39 | 35 |
| Gel elastic modulus ($\times 10^3$ N/m$^2$) | 2.3 | 2.4 | 2.6 | 2.6 | 2 | 2.3 | 2.3 | 2.4 | 2.2 | 1.9 | 2.5 | 2.1 | 2.7 | 2.6 | 2.2 | 2.7 | 2.3 | 2.2 | 2.2 | 2 |

<Examples 61 to 80>

According to the charge formulation in Table 2, an aqueous 48.5 wt% sodium hydroxide solution was added and mixed while mincing a hydrous gel of (B2) a crosslinked product of polyacrylic acid, an aqueous solution of (A1) polysaccharide oxide or a hydrous gel of (A2) a crosslinked product of polysaccharide oxide, and a binder solution by a mincer (12VR-400K manufactured by ROYAL Co.) at a temperature of 80°C to obtain a minced gel. The subsequent procedure was performed in the same manner as in Example 41 to obtain absorbent compositions of Examples 61 to 80. The measurement results of water-retention amount and gel elastic modulus of the obtained absorbent compositions are shown in Table 2. Here, in Table 2, the solution of surface crosslinking agent is a 2 wt% water/methanol (weight ratio = 70/30) mixed solution of ethylene glycol diglycidyl ether. The binder solution is a 2 wt% water/methanol (weight ratio = 70/30) mixed solution of ethylene glycol diglycidyl ether.

[Table 2]

| Table 2 | | Example | | | | | | | | | | | | | | | | | | |
|---|---|---|---|---|---|---|---|---|---|---|---|---|---|---|---|---|---|---|---|---|
| | | 61 | 62 | 63 | 64 | 65 | 66 | 67 | 68 | 69 | 70 | 71 | 72 | 73 | 74 | 75 | 76 | 77 | 78 | 79 | 80 |
| Hydrous gel (parts) of (B2) crosslinked product of polyacrylic acid | (B2-1) | 60 | 60 | 70 | 60 | 20 | 60 | 60 | 70 | 60 | 20 | 60 | 60 | 70 | 60 | 20 | 60 | 60 | 70 | 60 | 20 |
| Aqueous solution (parts) of (A1) polysaccharide oxide | (A1-1) | 40 | - | - | - | - | - | - | - | - | - | - | - | - | - | - | - | - | - | - | - |
| | (A1-2) | - | 40 | - | - | - | - | - | - | - | - | - | - | - | - | - | - | - | - | - | - |
| | (A1-3) | - | - | 30 | 40 | 80 | - | - | - | - | - | - | - | - | - | - | - | - | - | - | - |
| | (A1-4) | - | - | - | - | - | 40 | - | - | - | - | - | - | - | - | - | - | - | - | - | - |
| | (A1-5) | - | - | - | - | - | - | 40 | - | - | - | - | - | - | - | - | - | - | - | - | - |
| | (A1-6) | - | - | - | - | - | - | - | 30 | 40 | 80 | - | - | - | - | - | - | - | - | - | - |
| Hydrous gel (parts) of (A2) cross-linked product of polysaccharide oxide | (A2-1) | - | - | - | - | - | - | - | - | - | - | 40 | - | - | - | - | - | - | - | - | - |
| | (A2-2) | - | - | - | - | - | - | - | - | - | - | - | 40 | - | - | - | - | - | - | - | - |
| | (A2-3) | - | - | - | - | - | - | - | - | - | - | - | - | 30 | 40 | 80 | - | - | - | - | - |
| | (A2-4) | - | - | - | - | - | - | - | - | - | - | - | - | - | - | - | 40 | - | - | - | - |
| | (A2-5) | - | - | - | - | - | - | - | - | - | - | - | - | - | - | - | - | 40 | - | - | - |
| | (A2-6) | - | - | - | - | - | - | - | - | - | - | - | - | - | - | - | - | - | 30 | 40 | 80 |

| Table 2 | Example | | | | | | | | | | | | | | | | | | | |
|---|---|---|---|---|---|---|---|---|---|---|---|---|---|---|---|---|---|---|---|---|
| | 61 | 62 | 63 | 64 | 65 | 66 | 67 | 68 | 69 | 70 | 71 | 72 | 73 | 74 | 75 | 76 | 77 | 78 | 79 | 80 |
| Binder solution | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| Aqueous 48.5 wt% NaOH solution (parts) | 23.1 | 21.1 | 24.3 | 24.3 | 24.6 | 23.7 | 22.0 | 21.7 | 20.9 | 17.8 | 23.1 | 23.1 | 24.3 | 24.3 | 24.6 | 23.7 | 22.0 | 21.7 | 20.9 | 17.8 |
| Solution (parts) of surface crosslinking agent | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | 36 | 37 | 35 | 35 | 38 | 31 | 37 | 39 | 38 | 34 | 36 | 33 | 39 | 39 | 40 | 39 | 38 | 35 | 34 | 31 |
| Gel elastic modulus ($\times 10^3$ N/m$^2$) | 2.5 | 2.1 | 2.7 | 2.6 | 2.4 | 2.7 | 2.3 | 2.4 | 2.4 | 1.9 | 2.6 | 2.3 | 2.4 | 2.3 | 2 | 2.2 | 2.7 | 2.6 | 2.6 | 2.1 |

<Examples 81 to 100>

According to the charge formulation in Table 3, an aqueous 48.5 wt% sodium hydroxide solution was added and mixed while mincing an aqueous solution of (B1) polyacrylic acid and an aqueous solution of (A1) polysaccharide oxide or a hydrous gel of (A2) a crosslinked product of polysaccharide oxide by a mincer (12VR-400K manufactured by ROYAL Co.) to obtain a minced gel. The subsequent procedure was performed in the same manner as in Example 41 to obtain absorbent compositions of Examples 81 to 100. The measurement results of water-retention amount and gel elastic modulus of the obtained absorbent compositions are shown in Table 3. Here, in Table 3, the solution of surface crosslinking agent is a 2 wt% water/methanol (weight ratio = 70/30) mixed solution of ethylene glycol diglycidyl ether.

[Table 3]

| Table 3 | | Example | | | | | | | | | | | | | | | | | | |
|---|---|---|---|---|---|---|---|---|---|---|---|---|---|---|---|---|---|---|---|---|
| | | 81 | 82 | 83 | 84 | 85 | 86 | 87 | 88 | 89 | 90 | 91 | 92 | 93 | 94 | 95 | 96 | 97 | 98 | 99 | 100 |
| Aqueous solution (parts) of (B1) polyacrylic acid | (B1-1) | 60 | 60 | 70 | 60 | 20 | 60 | 60 | 70 | 60 | 20 | 60 | 60 | 70 | 60 | 20 | 60 | 60 | 70 | 60 | 20 |
| Aqueous solution (parts) of (A1) polysaccharide oxide | (A1-1) | 40 | - | - | - | - | - | - | - | - | - | - | - | - | - | - | - | - | - | - | - |
| | (A1-2) | - | 40 | - | - | - | - | - | - | - | - | - | - | - | - | - | - | - | - | - | - |
| | (A1-3) | - | - | 30 | 40 | 80 | - | - | - | - | - | - | - | - | - | - | - | - | - | - | - |
| | (A1-4) | - | - | - | - | - | 40 | - | - | - | - | - | - | - | - | - | - | - | - | - | - |
| | (A1-5) | - | - | - | - | - | - | 40 | - | - | - | - | - | - | - | - | - | - | - | - | - |
| | (A1-6) | - | - | - | - | - | - | - | 30 | 40 | 80 | - | - | - | - | - | - | - | - | - | - |
| Hydrous gel (parts) of (A2) cross-linked product of polysaccharide oxide | (A2-1) | - | - | - | - | - | - | - | - | - | - | 40 | - | - | - | - | - | - | - | - | - |
| | (A2-2) | - | - | - | - | - | - | - | - | - | - | - | 40 | - | - | - | - | - | - | - | - |
| | (A2-3) | - | - | - | - | - | - | - | - | - | - | - | - | 30 | 40 | 80 | - | - | - | - | - |
| | (A2-4) | - | - | - | - | - | - | - | - | - | - | - | - | - | - | - | 40 | - | - | - | - |
| | (A2-5) | - | - | - | - | - | - | - | - | - | - | - | - | - | - | - | - | 40 | - | - | - |
| | (A2-6) | - | - | - | - | - | - | - | - | - | - | - | - | - | - | - | - | - | 30 | 40 | 80 |

(continued)

| Table 3 | Example | | | | | | | | | | | | | | | | | | | |
|---|---|---|---|---|---|---|---|---|---|---|---|---|---|---|---|---|---|---|---|---|
| | 81 | 82 | 83 | 84 | 85 | 86 | 87 | 88 | 89 | 90 | 91 | 92 | 93 | 94 | 95 | 96 | 97 | 98 | 99 | 100 |
| Aqueous 48.5 wt% NaOH solution (parts) | 23.1 | 21.1 | 24.3 | 24.3 | 24.6 | 23.7 | 22.0 | 21.7 | 20.9 | 17.8 | 23.1 | 23.1 | 24.3 | 24.3 | 24.6 | 23.7 | 22.0 | 21.7 | 20.9 | 17.8 |
| Solution (parts) of surface crosslinking agent | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | 43 | 42 | 43 | 45 | 46 | 41 | 43 | 45 | 46 | 40 | 42 | 42 | 41 | 42 | 44 | 46 | 45 | 45 | 45 | 39 |
| Gel elastic modulus ($\times 10^3$ N/m$^2$) | 2.1 | 2.1 | 2.2 | 2 | 1.8 | 1.9 | 1.9 | 2 | 1.8 | 1.7 | 1.8 | 2.1 | 2.1 | 2 | 1.9 | 2 | 2.1 | 2.1 | 2.1 | 1.7 |

<Examples 101 to 120>

According to the charge formulation in Table 4, 23.1 parts of an aqueous 48.5 wt% sodium hydroxide solution was added and mixed while mincing an aqueous solution of (B1) polyacrylic acid, an aqueous solution of (A1) polysaccharide oxide or a hydrous gel of (A2) a crosslinked product of polysaccharide oxide, and a binder solution at a temperature of 80°C by a mincer (12VR-400K manufactured by ROYAL Co.) to obtain a minced gel. The subsequent procedure was performed in the same manner as in Example 41 to obtain absorbent compositions of Examples 101 to 120. The measurement results of water-retention amount and gel elastic modulus of the obtained absorbent compositions are shown in Table 4. Here, in Table 4, the solution of surface crosslinking agent is a 2 wt% water/methanol (weight ratio = 70/30) mixed solution of ethylene glycol diglycidyl ether. The binder solution is a 2 wt% water/methanol (weight ratio = 70/30) mixed solution of ethylene glycol diglycidyl ether.

[Table 4]

| Table 4 | | Example | | | | | | | | | | | | | | | | | | | |
|---|---|---|---|---|---|---|---|---|---|---|---|---|---|---|---|---|---|---|---|---|---|---|
| | | 101 | 102 | 103 | 104 | 105 | 106 | 107 | 108 | 109 | 110 | 111 | 112 | 113 | 114 | 115 | 116 | 117 | 118 | 119 | 120 |
| Aqueous solution (parts) of (B1) poly-acrylic acid | (B1-1) | 60 | 60 | 70 | 60 | 20 | 60 | 60 | 70 | 60 | 20 | 60 | 60 | 70 | 60 | 20 | 60 | 60 | 70 | 60 | 20 |
| Aqueous solution (parts) of (A1) Polysaccharide oxide | (A1-1) | 40 | - | - | - | - | - | - | - | - | - | - | - | - | - | - | - | - | - | - | - |
| | (A1-2) | - | 40 | - | - | - | - | - | - | - | - | - | - | - | - | - | - | - | - | - | - |
| | (A1-3) | - | - | 30 | 40 | 80 | - | - | - | - | - | - | - | - | - | - | - | - | - | - | - |
| | (A1-4) | - | - | - | - | - | 40 | - | - | - | - | - | - | - | - | - | - | - | - | - | - |
| | (A1-5) | - | - | - | - | - | - | 40 | - | - | - | - | - | - | - | - | - | - | - | - | - |
| | (A1-6) | - | - | - | - | - | - | - | 30 | 40 | 80 | - | - | - | - | - | - | - | - | - | - |
| Hydrous gel (parts) of (A2) crosslinked product of polysaccharide oxide | (A2-1) | - | - | - | - | - | - | - | - | - | - | 40 | - | - | - | - | - | - | - | - | - |
| | (A2-2) | - | - | - | - | - | - | - | - | - | - | - | 40 | - | - | - | - | - | - | - | - |
| | (A2-3) | - | - | - | - | - | - | - | - | - | - | - | - | 30 | 40 | 80 | - | - | - | - | - |
| | (A2-4) | - | - | - | - | - | - | - | - | - | - | - | - | - | - | - | 40 | - | - | - | - |
| | (A2-5) | - | - | - | - | - | - | - | - | - | - | - | - | - | - | - | - | 40 | - | - | - |
| | (A2-6) | - | - | - | - | - | - | - | - | - | - | - | - | - | - | - | - | - | 30 | 40 | 80 |

| Table 4 | Example | | | | | | | | | | | | | | | | | | | |
|---|---|---|---|---|---|---|---|---|---|---|---|---|---|---|---|---|---|---|---|---|
| | 101 | 102 | 103 | 104 | 105 | 106 | 107 | 108 | 109 | 110 | 111 | 112 | 113 | 114 | 115 | 116 | 117 | 118 | 119 | 120 |
| Binder solution | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| Aqueous 48.5 wt% NaOH solution (parts) | 23.1 | 21.1 | 24.3 | 24.3 | 24.6 | 23.7 | 22.0 | 21.7 | 20.9 | 17.8 | 23.1 | 23.1 | 24.3 | 24.3 | 24.6 | 23.7 | 22.0 | 21.7 | 20.9 | 17.8 |
| Solution (parts) of surface crosslinking agent | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | 40 | 38 | 40 | 42 | 43 | 38 | 41 | 40 | 40 | 35 | 43 | 41 | 38 | 38 | 40 | 37 | 38 | 38 | 38 | 36 |
| Gel elastic modulus ($\times 10^3$ N/m$^2$) | 2.2 | 2.5 | 2.2 | 2.1 | 1.9 | 2.2 | 2.3 | 2.1 | 2 | 1.7 | 1.9 | 1.9 | 2.2 | 2 | 2 | 2.3 | 2.1 | 2.2 | 2.2 | 2 |

<Examples 121 to 160>

An aqueous 48.5 wt% sodium hydroxide solution was added and mixed while mincing a hydrous gel of (G) graft polymer by a mincer (12VR-400K manufactured by ROYAL Co.) to obtain a minced gel. The subsequent procedure was performed in the same manner as in Example 41 to obtain absorbent compositions of Examples 121 to 160. The measurement results of water-retention amount and gel elastic modulus of the obtained absorbent compositions are shown in Tables 5-1 and 5-2. Here, in Tables 5-1 and 5-2, the solution of surface crosslinking agent is a 2 wt% water/methanol (weight ratio = 70/30) mixed solution of ethylene glycol diglycidyl ether.

[Table 5]

EP 2 690 133 A1

| Table 5-1 | | Example | | | | | | | | | | | | | | | | | | |
|---|---|---|---|---|---|---|---|---|---|---|---|---|---|---|---|---|---|---|---|---|
| | | 121 | 122 | 123 | 124 | 125 | 126 | 127 | 128 | 129 | 130 | 131 | 132 | 133 | 134 | 135 | 136 | 137 | 138 | 139 | 140 |
| Hydrous gel (parts) of (G) graft polymer | (G1) | 100 | - | - | - | - | - | - | - | - | - | - | - | - | - | - | - | - | - | - | - |
| | (G2) | - | 100 | - | - | - | - | - | - | - | - | - | - | - | - | - | - | - | - | - | - |
| | (G3) | - | - | 100 | - | - | - | - | - | - | - | - | - | - | - | - | - | - | - | - | - |
| | (G4) | - | - | - | 100 | - | - | - | - | - | - | - | - | - | - | - | - | - | - | - | - |
| | (G5) | - | - | - | - | 100 | - | - | - | - | - | - | - | - | - | - | - | - | - | - | - |
| | (G6) | - | - | - | - | - | 100 | - | - | - | - | - | - | - | - | - | - | - | - | - | - |
| | (G7) | - | - | - | - | - | - | 100 | - | - | - | - | - | - | - | - | - | - | - | - | - |
| | (G8) | - | - | - | - | - | - | - | 100 | - | - | - | - | - | - | - | - | - | - | - | - |
| | (G9) | - | - | - | - | - | - | - | - | 100 | - | - | - | - | - | - | - | - | - | - | - |
| | (G10) | - | - | - | - | - | - | - | - | - | 100 | - | - | - | - | - | - | - | - | - | - |
| | (G11) | - | - | - | - | - | - | - | - | - | - | 100 | - | - | - | - | - | - | - | - | - |
| | (G12) | - | - | - | - | - | - | - | - | - | - | - | 100 | - | - | - | - | - | - | - | - |
| | (G13) | - | - | - | - | - | - | - | - | - | - | - | - | 100 | - | - | - | - | - | - | - |
| | (G14) | - | - | - | - | - | - | - | - | - | - | - | - | - | 100 | - | - | - | - | - | - |
| | (G15) | - | - | - | - | - | - | - | - | - | - | - | - | - | - | 100 | - | - | - | - | - |
| | (G16) | - | - | - | - | - | - | - | - | - | - | - | - | - | - | - | 100 | - | - | - | - |
| | (G17) | - | - | - | - | - | - | - | - | - | - | - | - | - | - | - | - | 100 | - | - | - |
| | (G18) | - | - | - | - | - | - | - | - | - | - | - | - | - | - | - | - | - | 100 | - | - |
| | (G19) | - | - | - | - | - | - | - | - | - | - | - | - | - | - | - | - | - | - | 100 | - |
| | (G20) | - | - | - | - | - | - | - | - | - | - | - | - | - | - | - | - | - | - | - | 100 |
| Aqueous 48.5 wt% NaOH solution (parts) | | 23.1 | 21.1 | 24.3 | 24.3 | 24.6 | 23.7 | 22.0 | 21.7 | 20.9 | 17.8 | 23.1 | 23.1 | 24.3 | 24.3 | 24.6 | 23.7 | 22.0 | 21.7 | 20.9 | 17.8 |

(continued)

| Table 5-1 | Example | | | | | | | | | | | | | | | | | | | |
|---|---|---|---|---|---|---|---|---|---|---|---|---|---|---|---|---|---|---|---|
| | 121 | 122 | 123 | 124 | 125 | 126 | 127 | 128 | 129 | 130 | 131 | 132 | 133 | 134 | 135 | 136 | 137 | 138 | 139 | 140 |
| Solution (parts) of surface crosslinking agent | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | 37 | 31 | 38 | 38 | 40 | 39 | 38 | 35 | 34 | 33 | 42 | 41 | 40 | 40 | 43 | 43 | 37 | 39 | 38 | 33 |
| Gel elastic modulus ($\times 10^3$ N/m$^2$) | 2.3 | 2.1 | 2.2 | 2.2 | 2.1 | 2.2 | 2.3 | 2.4 | 2.4 | 2 | 2 | 2 | 2 | 1.9 | 1.7 | 1.9 | 2.1 | 2 | 2 | 1.8 |

[Table 6]

| Table 5-2 | | Example | | | | | | | | | | | | | | | | | | | |
|---|---|---|---|---|---|---|---|---|---|---|---|---|---|---|---|---|---|---|---|---|---|
| | | 141 | 142 | 143 | 144 | 145 | 146 | 147 | 148 | 149 | 150 | 151 | 152 | 153 | 154 | 155 | 156 | 157 | 158 | 159 | 160 |
| Hydrous gel (parts) of (G) graft polymer | (G21) | 100 | - | - | - | - | - | - | - | - | - | - | - | - | - | - | - | - | - | - | - |
| | (G22) | - | 100 | - | - | - | - | - | - | - | - | - | - | - | - | - | - | - | - | - | - |
| | (G23) | - | - | 100 | - | - | - | - | - | - | - | - | - | - | - | - | - | - | - | - | - |
| | (G24) | - | - | - | 100 | - | - | - | - | - | - | - | - | - | - | - | - | - | - | - | - |
| | (G25) | - | - | - | - | 100 | - | - | - | - | - | - | - | - | - | - | - | - | - | - | - |
| | (G26) | - | - | - | - | - | 100 | - | - | - | - | - | - | - | - | - | - | - | - | - | - |
| | (G27) | - | - | - | - | - | - | 100 | - | - | - | - | - | - | - | - | - | - | - | - | - |
| | (G28) | - | - | - | - | - | - | - | 100 | - | - | - | - | - | - | - | - | - | - | - | - |
| | (G29) | - | - | - | - | - | - | - | - | 100 | - | - | - | - | - | - | - | - | - | - | - |
| | (G30) | - | - | - | - | - | - | - | - | - | 100 | - | - | - | - | - | - | - | - | - | - |
| | (G31) | - | - | - | - | - | - | - | - | - | - | 100 | - | - | - | - | - | - | - | - | - |
| | (G32) | - | - | - | - | - | - | - | - | - | - | - | 100 | - | - | - | - | - | - | - | - |
| | (G33) | - | - | - | - | - | - | - | - | - | - | - | - | 100 | - | - | - | - | - | - | - |
| | (G39) | - | - | - | - | - | - | - | - | - | - | - | - | - | 100 | - | - | - | - | - | - |
| | (G35) | - | - | - | - | - | - | - | - | - | - | - | - | - | - | 100 | - | - | - | - | - |
| | (G36) | - | - | - | - | - | - | - | - | - | - | - | - | - | - | - | 100 | - | - | - | - |
| | (G37) | - | - | - | - | - | - | - | - | - | - | - | - | - | - | - | - | 100 | - | - | - |
| | (G38) | - | - | - | - | - | - | - | - | - | - | - | - | - | - | - | - | - | 100 | - | - |
| | (G39) | - | - | - | - | - | - | - | - | - | - | - | - | - | - | - | - | - | - | 100 | - |
| | (G40) | - | - | - | - | - | - | - | - | - | - | - | - | - | - | - | - | - | - | - | 100 |
| Aqueous 48.5 wt% NaOH solution (parts) | | 23.1 | 23.1 | 21.1 | 24.3 | 24.3 | 24.6 | 23.7 | 22.0 | 21.7 | 20.9 | 17.8 | 23.1 | 23.1 | 24.3 | 24.3 | 24.6 | 23.7 | 22.0 | 21.7 | 20.9 |

| Table 5-2 | Example | | | | | | | | | | | | | | | | | | | |
|---|---|---|---|---|---|---|---|---|---|---|---|---|---|---|---|---|---|---|---|---|
| | 141 | 142 | 143 | 144 | 145 | 146 | 147 | 148 | 149 | 150 | 151 | 152 | 153 | 154 | 155 | 156 | 157 | 158 | 159 | 160 |
| Solution (parts) of surface crosslinking agent | 5 | 5 | 5 | 5 | 5 | 5 | 5 | 5 | 5 | 5 | 5 | 5 | 5 | 5 | 5 | 5 | 5 | 5 | 5 | 5 |
| Water-retention amount (g/g) | 37 | 37 | 39 | 38 | 39 | 42 | 36 | 38 | 35 | 35 | 30 | 43 | 41 | 41 | 42 | 46 | 42 | 42 | 45 | 45 |
| Gel elastic modulus ($\times 10^3$ N/m$^2$) | 2.3 | 2 | 2.1 | 2.1 | 2 | 1.7 | 1.7 | 2.1 | 2.2 | 2.2 | 2 | 1.9 | 1.9 | 2.1 | 2 | 1.6 | 2 | 1.9 | 2 | 2 |

<Comparative Example 1>

An adsorbent composition for comparison was obtained in the same manner as in Example 41 except for using a 30 wt% aqueous solution of commercially available starch (SK-100 produced by Japan Corn Starch Co., Ltd.) in place of Aqueous Solution (A1-1) of polysaccharide oxide and changing the charge amount of an aqueous 48.5 wt% sodium hydroxide solution to 16.8 parts.

<Comparative Example 2>

An adsorbent composition for comparison was obtained in the same manner as in Example 41 except for using a 30 wt% aqueous solution of commercially available starch (produced by Wako Pure Chemical Industries, Ltd.) in place of Aqueous Solution (A1-1) of polysaccharide oxide and changing the charge amount of an aqueous 48.5 wt% sodium hydroxide solution to 16.8 parts.

<Comparative Example 3>

An adsorbent composition for comparison was obtained in the same manner as in Example 41 except for using a 30 wt% aqueous solution of commercially available Kiprogum (M-800A, produced by Nippon Starch Chemical Co., Ltd.) in place of Aqueous Solution (A1-1) of polysaccharide oxide and changing the charge amount of an aqueous 48.5 wt% sodium hydroxide solution to 16.8 parts.

<Comparative Example 4>

An adsorbent composition for comparison was obtained in the same manner as in Example 61 except for using a 30 wt% aqueous solution of commercially available carboxymethyl cellulose (produced by Aldrich Chemical Co. Inc., substitution degree: 0.7) in place of Aqueous Solution (A1-1) of polysaccharide oxide and changing the charge amount of an aqueous 48.5 wt% sodium hydroxide solution to 20.5 parts.

<Comparative Example 5>

An adsorbent composition for comparison was obtained in the same manner as in Example 61 except for using 100 parts of a 30 wt% aqueous solution of commercially available carboxymethyl cellulose (produced by Aldrich Chemical Co. Inc., substitution degree: 0.7) in place of 60 parts of Hydrous Gel (B2-1) of crosslinked polyacrylic acid and 40 parts of Aqueous Solution (A1-1) of polysaccharide oxide and changing the charge amount of an aqueous 48.5 wt% sodium hydroxide solution to 12.2 parts.

<Comparative Example 6>

An adsorbent composition for comparison was obtained in the same manner as in Example 41 except for using 100 parts of a 30 wt% aqueous solution of commercially available Kiprogum (M-800A, produced by Nippon Starch Chemical Co., Ltd.) in place of 60 parts of Hydrous Gel (B2-1) of crosslinked polyacrylic acid and 40 parts of Aqueous Solution (A1-1) of polysaccharide oxide and changing the charge amount of an aqueous 48.5 wt% sodium hydroxide solution to 2.2 parts.

The measurement results of water-retention amount and gel elastic modulus of the absorbent compositions for comparison obtained in Comparative Examples 1 to 6 are shown in Table 6.

[Table 7]

| Table 6 | | Water-Retention Amount (g/g) | Gel Elastic Modulus ($\times 10^3$ N/m$^2$) |
|---|---|---|---|
| Comparative Example | 1 | 22 | 1.6 |
| | 2 | 19 | 1.6 |
| | 3 | 23 | 1.7 |
| | 4 | 26 | 1.6 |
| | 5 | 14 | 2.8 |
| | 6 | 8 | 1.0 |

It is seen from Tables 1 to 6 that as compared with absorbent compositions of Comparative Examples 1 to 6, the absorbent compositions of Examples 1 to 160 are excellent in the water-retention amount. Also, as compared with absorbent compositions of Comparative Examples 5 and 6, which are composed of only a polysaccharide having a carboxyl group, the absorbent compositions of Examples 1 to 160 has high gel elastic modulus.

<Example 161>

24.3 Parts of an aqueous 48.5 wt% sodium hydroxide solution was added and mixed while mincing 60 parts of Hydrous Gel (B2-1) of crosslinked polyacrylic acid obtained in Production Example 1 and 40 parts of Aqueous Solution (A1-1) of polysaccharide oxide obtained in Production Example 3 by a mincer (12VR-400K manufactured by ROYAL Co.), and subsequently, 0.19 parts of octadecanoic acid as a hydrophobic substance was added and mixed to obtain a minced gel. The minced gel was dried in a through-flow band-type dryer (150°C, wind velocity: 2 m/sec) to obtain a dry form. The dry form was pulverized in a juicer-mixer (OSTERIZER BLENDER, manufactured by Oster Co.) and then adjusted to a particle size of 150 to 710 μm by using sieves with a sieve-opening of 150 and 710 μm, whereby dry form particles were obtained. While stirring 100 parts of the dry form particles at a high speed (high-speed stirring turbulizer manufactured by Hosokawa Micron Corporation; rotation speed: 2,000 rpm), 5 parts of a 2 wt% water/methanol mixed solution (weight ratio of water/methanol = 70/30) of ethylene glycol diglycidyl ether was added and mixed by spraying, and the system was allowed to stand at 150°C for 30 minutes so as to effect surface crosslinking, whereby an absorbent composition particle was obtained. The hydrophobic substance (octadecanoic acid) is present in a proportion of 0.520% in the inside of the absorbent composition particle and present in a proportion of 0.015% on the surface.

<Example 162>

An absorbent composition particle was obtained in the same manner as in Example 161 except for adding 0.38 parts of sucrose mononononanoate in place of 0.19 parts of octadecanoic acid. The hydrophobic substance (sucrose mononononanoate) was present in a proportion of 1.03% in the inside of the absorbent composition particle and present in a proportion of 0.061% on the surface.

<Example 163>

An absorbent composition particle of the present invention was obtained in the same manner as in Example 161 except for adding 0.38 parts of sorbitol mononononanoate in place of 0.19 parts of octadecanoic acid. The hydrophobic substance (sorbitol mononononanoate) was present in a proportion of 1.05% in the inside of the absorbent composition particle and present in a proportion of 0.035% on the surface.

<Comparative Example 7>

An absorbent composition particle for comparison was obtained in the same manner as in Example 161 except for not using Aqueous Solution (A1-1) of polysaccharide oxide and octadecanoic acid and changing the parts used of Hydrous Gel (B2-1) of crosslinked polyacrylic acid to 100 parts and the parts used of an aqueous 48.5 wt% sodium hydroxide solution to 24.0 parts.

The absorbent composition particles obtained in Examples 161 to 163 and Comparative Example 7 were measured for the water-retention amount, the absorption rate by a swelled volume measuring method, and the gel elastic modulus, and the results are shown in Table 7.

[Table 8]

| Table 7 | | Content of Hydrophobic Substance (wt%) | | Water-Retention Amount (g/g) | Absorption Rate by Swelled Volume Measuring Method | | | Gel Elastic Modulus ($\times 10^3$ N/m$^2$) |
|---|---|---|---|---|---|---|---|---|
| | | Inside of Particle | Surface of Particle | | t1 (sec) | t2 (sec) | t2/t1 | |
| Example | 161 | 0.52 | 0.015 | 35 | 31 | 290 | 9.4 | 2.6 |
| | 162 | 1.03 | 0.061 | 37 | 31 | 336 | 10.8 | 2.4 |
| | 163 | 1.05 | 0.035 | 37 | 39 | 331 | 8.5 | 2.6 |
| Comparative Example | 7 | 0 | 0 | 36 | 19 | 577 | 30.4 | 2.4 |

It is seen from Table 7 that as compared with the absorbent composition particle of Comparative Example 7, the absorbent composition particles of Examples 161 to 163 have a fast absorption rate in the middle and late stages and are an absorbent composition particle having an absorption rate pattern more suitable for an absorbent article.

<Examples 164 to 166 and Comparative Example 8>

In order to examine the absorption characteristics when an absorbent composition particle having an appropriate absorption rate pattern is applied to an absorbent article, two kinds of absorbent articles (disposable diaper) were prepared as follows by using each of the absorbent composition particles obtained in Examples 161 to 163 and Comparative Example 7, and the surface dryness value was evaluated by the SDME method. The results are shown in Table 8.

<Preparation 1 of Absorbent Article (Disposable Diaper)>

100 Parts of fluff pulp and 100 parts of the evaluation sample (absorbent composition particle) were mixed in an air-flow type mixing apparatus (pad former manufactured by O-TEC K.K.) to obtain a mixture, and this mixture was uniformly laminated on an acryl plate (thickness: 4 mm) to have a basis weight of about 500 $g/m^2$ and then pressed under a pressure of 5 $kg/cm^2$ for 30 seconds to obtain Absorber (1). Absorber (1) was cut into a rectangle of 10 cm$\times$40 cm and after a water-absorbing paper (basis weight: 15.5 $g/m^2$, produced by Advantec Co., filter paper No. 2) having the same size as that of the absorber was disposed on the top and the bottom of each absorber, a polyethylene sheet (polyethylene film UB-1 produced by Tamapoly Co., Ltd.) and a nonwoven fabric (basis weight: 20 $g/m^2$, Eltas Guard produced by Asahi Kasei Corporation) were disposed on the back side and the surface, respectively, to prepare Disposable Diaper (1). The weight ratio between the absorbent composition particle and the fiber (weight of absorbent composition particle/weight of fiber) was 50/50.

<Preparation 2 of Absorbent Article (Disposable Diaper)>

Disposable Diaper (2) was prepared in the same manner as in Preparation 1 of Absorbent Article (Disposable Diaper) except for changing "100 parts of fluff pulp and 100 parts of the evaluation sample (absorbent composition particle)" to "80 parts of fluff pulp and 120 parts of the evaluation sample (absorbent composition particle)". The weight ratio between the absorbent composition particle and the fiber (weight of absorbent composition particle/weight of fiber) was 60/40.

<Surface Dryness Value by SDME Method>

A detector of an SDME (Surface Dryness Measurement Equipment) tester (manufactured by WK system Co.) was placed on a fully wetted disposable diaper [prepared by dipping a disposable diaper in an artificial urine (0.03 wt% of potassium chloride, 0.08 wt% of magnesium sulfate, 0.8 wt% of sodium chloride, and 99.09 wt% of deionized water) and allowing the diaper to stand for 60 minutes] to set a 0% dryness value and then, the detector of the SDME tester was placed on a dry disposable diaper (prepared by drying a disposable diaper under heating at 80°C for 2 hours) to set a 100% dryness value, thereby performing calibration of the SDME tester. Subsequently, a metal ring (inner diameter: 70 mm, length: 50 mm) was set on the center of the disposable diaper to be measured, and 80 ml of the artificial urine was poured therein. When absorption of the artificial urine was completed (until the gloss by the artificial urine was not confirmed), the metal ring was immediately removed and by placing three SDME detectors at the center, right side and left side of the disposable diaper (3 positions at equal intervals of 10 cm from the end of the 40 cm-long disposable diaper), measurement of the surface dryness value was started. The values 5 minutes after the start of measurement were taken as the surface dryness value (center), the surface dryness value (left), and the surface dryness value (right).
Incidentally, the measurement was performed by setting the artificial urine, the measurement atmosphere and the standing atmosphere at 25±5°C and 65±10% RH.

[Table 9]

| Table 8 | | Disposable Diaper (1) | | | Disposable Diaper (2) | | |
| --- | --- | --- | --- | --- | --- | --- | --- |
| | | Surface Dryness Value (%) | | | Surface Dryness Value (%) | | |
| | | (Left) | (Center) | (Right) | (Left) | (Center) | (Right) |
| Example | 164 | 84 | 89 | 86 | 90 | 91 | 93 |
| | 165 | 86 | 90 | 88 | 79 | 87 | 80 |
| | 166 | 90 | 90 | 83 | 85 | 79 | 89 |
| Comparative Example | 8 | 79 | 64 | 65 | 80 | 65 | 60 |

As seen from Table 8, the disposable diapers using the absorbent composition particles of Examples 161 to 163 were excellent with less variation among the surface dryness values (center), (left) and (right), as compared with the disposable diaper using the absorbent composition particle of Comparative Example 7. That is, thanks to a more appropriate absorption rate pattern, the absorbent composition particle of the present invention exhibited excellent absorption characteristics when applied to an absorbent article. Accordingly, it may be easily expected that even when an absorbent article to which the absorbent composition particle of the present invention is applied is used, there is no fear of skin irritation and the like.

INDUSTRIAL APPLICABILITY

The absorbent resin, absorbent resin particle, absorbent composition and absorbent composition particle of the present invention have a high plant-derived raw material proportion and are excellent in the aqueous liquid absorption ability and by virtue of having good gel elastic modulus, also excellent in the absorption ability in a pressurized state, and therefore, these are useful for various industrial applications such as hygienic material (e.g., disposable diaper for child and adult, sanitary napkin, hygienic cotton, bandage, incontinence pad, paper towel), a food material (e.g., freshness-keeping agent, drip absorbing agent), a garden material (e.g., water retention agent, soil conditioner), and a building material (e.g., anti-dewing agent).

DESCRIPTION OF REFERENCE NUMERALS AND SIGNS

1:    Bottomed cylinder
2:    Disk
3:    Absorbent composition particle
4:    Indication part for thickness measurement of Digimatic indicator
5:    Rod of Digimatic indicator
6:    Platform of Digimatic indicator

**Claims**

1.  An aqueous liquid absorbent resin formed by binding (A1) a polysaccharide oxide having a carboxyl group which may be neutralized with a neutralizer, and/or (A2) a crosslinked product thereof, to (B1) a poly(meth)acrylic acid in which at least one carboxyl group may be neutralized with a neutralizer, and/or (B2) a crosslinked product thereof, wherein a weight average molecular weight of said (A1) is from 2,000 to 10,000,000, an acid value of said (A1) is from 65 to 850 mgKOH/g when said (A1) does not have a carboxyl group neutralized with a neutralizer, and an acid value of said (A1) before neutralization is from 65 to 850 mgKOH/g when said (A1) has a carboxyl group neutralized with a neutralizer.

2.  The aqueous liquid absorbent resin as claimed in claim 1, wherein said (A1) is a starch oxide or cellulose oxide, which have a carboxyl group which may be neutralized with a neutralizer.

3.  The aqueous liquid absorbent resin as claimed in claim 1 or 2, wherein said (A1) and/or said (A2) is bound to said (B1) and/or said (B2) using at least one binder selected from the group consisting of (k1) a polyhydric alcohol having

41

a carbon number of 2 to 6, (k2) a polyvalent glycidyl ether having a carbon number of 8 to 21, (k3) a polyvalent amine having a carbon number of 2 to 6, and (k4) an alkanolamine having a carbon number of 2 to 8.

4.  The aqueous liquid absorbent resin as claimed in claim 1 or 2, wherein said (A1) and/or said (A2) is bound to said (B1) and/or said (B2) by graft-polymerizing said (B1) and/or said (B2) to said (A1) and/or said (A2).

5.  The aqueous liquid absorbent resin as claimed in any one of claims 1 to 4, wherein the neutralization ratio in said (B1) and said (B2) is less than 85% based on the total amount of carboxyl groups contained in said (B1) and said (B2).

6.  The aqueous liquid absorbent resin as claimed in any one of claims 1 to 5, wherein the neutralization ratio in said (A1), said (A2), said (B1) and said (B2) is from 65 to 75% based on the total amount of carboxyl groups contained in said (A1), said (A2), said (B1) and said (B2).

7.  The aqueous liquid absorbent resin as claimed in any one of claims 1 to 6, wherein said neutralizer is a hydroxide of an alkali metal.

8.  The aqueous liquid absorbent resin as claimed in any one of claims 1 to 7, wherein the total amount of said (A1) and (A2) bound is from 30 to 90 wt% based on the weight of the absorbent resin.

9.  An absorbent resin particle comprising the aqueous liquid absorbent resin claimed in any one of claims 1 to 8 and (E) a hydrophobic substance, wherein said (E) is a compound having at least one monovalent aliphatic hydrocarbon group with a carbon number of 8 to 26 and having at least one functional group capable of forming a hydrogen bond with a carboxyl group, and based on the weight of the aqueous liquid absorbent resin, said (E) is present in an amount of 0.01 to 10.0 wt% in the inside of the absorbent resin particle and present in an amount of 0.001 to 1.0 wt% on the surface of the absorbent resin particle.

10. The absorbent resin particle as claimed in claim 9, wherein when 1 g of the absorbent resin particle absorbs physiological saline at 25°C and swells, a ratio (t2/t1) between a time (t1) until the swelled volume reaches 5 ml and a time (t2) until the swelled volume reaches 40 ml, is from 5 to 20.

11. The absorbent resin particle as claimed in claim 9 or 10, wherein said functional group capable of forming a hydrogen bond with a carboxyl group is at least one functional group selected from the group consisting of a carboxyl group, a phosphoric acid group, a sulfonic acid group, a primary amino group, a secondary amino group, a tertiary amino group, a hydroxyl group, an oxycarbonyl group, a phosphoric acid ester group, a sulfonic acid ester group, an amide group, a urethane group, and a urea group.

12. An absorber formed using the aqueous liquid absorbent resin claimed in any one of claims 1 to 8 and/or the absorbent resin particle claimed in any one of claims 9 to 11, and at least one member selected from the group consisting of a fiber, a nonwoven fabric and a woven fabric.

13. An absorbent article formed using the absorber claimed in claim 12.

14. An aqueous liquid absorbent composition comprising: (A1) a polysaccharide oxide having a carboxyl group which may be neutralized with a neutralizer, and/or (A2) a crosslinked product thereof; and (B1) a poly(meth)acrylic acid in which at least one carboxyl group may be neutralized with a neutralizer, and/or (B2) a crosslinked product thereof, wherein a weight average molecular weight of said (A1) is from 2,000 to 10,000,000, an acid value of said (A1) is from 65 to 850 mgKOH/g when said (A1) does not have a carboxyl group neutralized with a neutralizer, and an acid value of said (A1) before neutralization is from 65 to 850 mgKOH/g when said (A1) has a carboxyl group neutralized with a neutralizer.

15. The aqueous liquid absorbent composition as claimed in claim 14, further comprising the aqueous liquid absorbent resin claimed in any one of claims 1 to 8.

16. The aqueous liquid absorbent composition as claimed in claim 14 or 15, wherein said (A1) is a starch oxide or cellulose oxide, which have a carboxyl group which may be neutralized with a neutralizer.

17. The aqueous liquid absorbent composition as claimed in any one of claims 14 to 16, wherein the neutralization ratio in said (B1) and said (B2) is less than 85% based on the total amount of carboxyl groups contained in said (B1) and said (B2).

18. The aqueous liquid absorbent composition as claimed in any one of claims 14 to 17, wherein the neutralization ratio in said (A1), said (A2), said (B1) and said (B2) is from 65 to 75% based on the total amount of carboxyl groups contained in said (A1), said (A2), said (B1) and said (B2).

19. The aqueous liquid absorbent composition as claimed in any one of claims 14 to 18, wherein said neutralizer is a hydroxide of an alkali metal.

20. The aqueous liquid absorbent composition as claimed in any one of claims 14 to 19, wherein when not containing the aqueous liquid absorbent resin, the total amount of said (A1) and said (A2) contained is from 30 to 90 wt% based on the weight of the aqueous liquid absorbent composition, and when containing the aqueous liquid absorbent resin, the total amount of said (A1) and said (A2) contained and said (A1) and said (A2) contained in the aqueous liquid absorbent resin is from 30 to 90 wt% based on the weight of the aqueous liquid absorbent composition.

21. An absorbent composition particle comprising: the aqueous liquid absorbent composition claimed in any one of claims 14 to 20; and (E) a hydrophobic substance, wherein said (E) is a compound having at least one monovalent aliphatic hydrocarbon group with a carbon number of 8 to 26 and having at least one functional group capable of forming a hydrogen bond with a carboxyl group, and based on the weight of the absorbent composition particle, said (E) is present in an amount of 0.01 to 10.0 wt% in the inside of the absorbent composition particle and present in an amount of 0.001 to 1.0 wt% on the surface of the absorbent composition particle.

22. The absorbent composition particle as claimed in claim 21, wherein when 1 g of the absorbent composition particle absorbs physiological saline at 25°C and swells, a ratio (t2/t1) between a time (t1) until the swelled volume reaches 5 ml and a time (t2) until the swelled volume reaches 40 ml, is from 5 to 20.

23. The absorbent composition particle as claimed in claim 21 or 22, wherein said functional group capable of forming a hydrogen bond with a carboxyl group is at least one functional group selected from the group consisting of a carboxyl group, a phosphoric acid group, a sulfonic acid group, a primary amino group, a secondary amino group, a tertiary amino group, a hydroxyl group, an oxycarbonyl group, a phosphoric acid ester group, a sulfonic acid ester group, an amide group, a urethane group, and a urea group.

24. An absorber formed using the aqueous liquid absorbent composition claimed in any one of claims 14 to 20 and/or the absorbent composition particle claimed in any one of claims 21 to 23, and at least one member selected from the group consisting of a fiber, a nonwoven fabric and a woven fabric.

25. An absorbent article formed using the absorber claimed in claim 24.

Fig. 1

## INTERNATIONAL SEARCH REPORT

| | International application No. |
|---|---|
| | PCT/JP2012/057089 |

**A. CLASSIFICATION OF SUBJECT MATTER**
*C08L3/10*(2006.01)i, *C08G81/00*(2006.01)i, *C08L33/04*(2006.01)i

According to International Patent Classification (IPC) or to both national classification and IPC

**B. FIELDS SEARCHED**

Minimum documentation searched (classification system followed by classification symbols)
C08L3/00-3/20, C08G81/00-81/02, C08L33/00-33/26

Documentation searched other than minimum documentation to the extent that such documents are included in the fields searched

| | | | |
|---|---|---|---|
| Jitsuyo Shinan Koho | 1922–1996 | Jitsuyo Shinan Toroku Koho | 1996–2012 |
| Kokai Jitsuyo Shinan Koho | 1971–2012 | Toroku Jitsuyo Shinan Koho | 1994–2012 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | JP 2008-303319 A (San-Dia Polymers, Inc.), 18 December 2008 (18.12.2008), entire text (Family: none) | 1-25 |
| A | JP 52-82715 A (Sanyo Chemical Industries, Ltd.), 11 July 1977 (11.07.1977), entire text (Family: none) | 1-25 |
| A | JP 53-149545 A (Sanyo Chemical Industries, Ltd., Sumitomo Forestry Co., Ltd.), 27 December 1978 (27.12.1978), entire text (Family: none) | 1-25 |

☒ Further documents are listed in the continuation of Box C.　☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 01 June, 2012 (01.06.12) | 12 June, 2012 (12.06.12) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| | International application No. |
| --- | --- |
| | PCT/JP2012/057089 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 51-125683 A  (Sanyo Chemical Industries, Ltd.),<br>02 November 1976 (02.11.1976),<br>entire text<br>(Family: none) | 1-25 |
| A | JP 60-4583 A  (Toppan Printing Co., Ltd.),<br>11 January 1985 (11.01.1985),<br>entire text<br>(Family: none) | 1-25 |
| A | JP 61-7316 A  (Sanyo Electric Co., Ltd., Tokyo Sanyo Electric Co., Ltd.),<br>14 January 1986 (14.01.1986),<br>entire text<br>(Family: none) | 1-25 |
| A | JP 3-174414 A  (Sanyo Chemical Industries, Ltd., Hoechst Celanese Corp.),<br>29 July 1991 (29.07.1991),<br>entire text<br>& JP 10-265522 A        & US 5453323 A<br>& US 5145906 A | 1-25 |
| A | JP 3-177431 A  (Sanyo Chemical Industries, Ltd.),<br>01 August 1991 (01.08.1991),<br>entire text<br>& US 5112903 A        & EP 407147 A2<br>& DE 69030844 T | 1-25 |
| A | JP 1-285119 A  (Sanyo Chemical Industries, Ltd.),<br>16 November 1989 (16.11.1989),<br>entire text<br>(Family: none) | 1-25 |
| A | JP 2007-270152 A  (Stockhausen GmbH),<br>18 October 2007 (18.10.2007),<br>entire text<br>(Family: none) | 1-25 |

Form PCT/ISA/210 (continuation of second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0489424 A
- JP 62247837 A
- JP 10195101 A
- JP 3205168 B
- JP 2003225565 A
- JP 2006131767 A
- JP 59189103 A
- JP 61211305 A
- JP 61252212 A
- JP 51136588 A
- JP 61257235 A
- JP 2011252088 A

**Non-patent literature cited in the description**

- **MASUDA.** *Chemical Economy & Engineering Review,* November 1983, 19
- Perrys Chemical Engineer's Handbook. Mac-Graw-Hill Book Company, 1984, 21